(19) Europäisches Patentamt
European Patent Office
Office européen des brevets

(11) **EP 2 145 631 B1**

(12) **EUROPEAN PATENT SPECIFICATION**

(45) Date of publication and mention
of the grant of the patent:
**05.09.2012 Bulletin 2012/36**

(51) Int Cl.:
*F24F 3/16* (2006.01)    *A61L 9/16* (2006.01)
*A61L 9/12* (2006.01)    *F24F 3/14* (2006.01)
*F24F 6/04* (2006.01)

(21) Application number: **09008973.1**

(22) Date of filing: **09.07.2009**

(54) **Air filtering apparatus**

Gerät zur Filtration von Luft

Dispositif de filtrage d'air

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.07.2008 JP 2008191721**
**11.07.2008 JP 2008181851**
**05.08.2008 JP 2008201671**

(43) Date of publication of application:
**20.01.2010 Bulletin 2010/03**

(73) Proprietor: **SANYO Electric Co., Ltd.**
**Moriguchi-shi**
**Osaka 560-8677 (JP)**

(72) Inventors:
• **Yamamoto, Tetsuya**
**Moriguchi-shi**
**Osaka 570-8677 (JP)**
• **Uchida, Yoichi**
**Moriguchi-shi**
**Osaka 570-8677 (JP)**
• **Rakuma, Tsuyoshi**
**Moriguchi-shi**
**Osaka 570-8677 (JP)**
• **Dobashi, Mitsuhiro**
**Moriguchi-shi**
**Osaka 570-8677 (JP)**
• **Ogawa, Yui**
**Moriguchi-shi**
**Osaka 570-8677 (JP)**
• **Umezawa, Hiroyuki**
**Moriguchi-shi**
**Osaka 570-8677 (JP)**
• **Kobayashi, Hiroyuki**
**Moriguchi-shi**
**Osaka 570-8677 (JP)**
• **Arakawa, Toru**
**Moriguchi-shi**
**Osaka 570-8677 (JP)**

(74) Representative: **Glawe, Delfs, Moll**
**Patent- und Rechtsanwälte**
**Postfach 26 01 62**
**80058 München (DE)**

(56) References cited:
**EP-A- 1 891 982        EP-A- 1 909 045**
**JP-A- 2003 250 876     US-A1- 2008 098 899**
**US-A1- 2008 115 925**

**Description**

BACKGROUND OF THE INVENTION

1. Field of the Invention

The present invention relates to an air filtering apparatus that can remove microorganisms floating in the air such as bacteria, virus, fungus, etc. (hereinafter referred to as "virus, etc.").

2. Description of the Related Art

There has been proposed an air filtering apparatus in which tap water is electrolyzed to generate electrolytic water containing hypochlorous acid and virus, etc. floating in the air are removed by using this electrolytic water (see JP-A-2002-181358 or JP-A-2007-175140, for example). In this air filtering apparatus, electrolytic water is supplied to a humidifying element formed of non-woven cloth or the like, and virus, etc. floating in the air are brought into contact with the electrolytic water on the humidifying element to inactivate the virus, etc., thereby filtering the air.

The electrolysis described above is performed by using chlorine ions contained in tap water. However, tap water in some districts where the air filtering apparatus is used contains a low concentration of chlorine and thus it is difficult to perform electrolysis in these districts. Accordingly, a method of performing electrolysis by making a larger amount of current flow through an electrolytic unit has been hitherto known. However, this method has a problem that a load imposed on electrodes is larger and thus the electrodes must be frequently exchanged in a maintenance work. Furthermore, there is known a method of supplying salt to tap water to be supplied for electrolysis. However, this is a cumbersome work because a user must supply salt every time the apparatus is operated.

In the air filtering apparatus described above, electrolytic water is used while circulated. However, when electrolytic water is circulated for a relatively long term, calcium or magnesium contained in electrolytic water is condensed due to vaporization, etc. of electrolytic water and thus the hardness of the electrolytic water increases. In connection with the increase of the hardness of the electrolytic water, scale such as calcium carbonate, magnesium carbonate, etc. may be easily deposited in the gas-liquid contact member, etc., for example.

EP-A-1 891 982 discloses an air filtering apparatus in which brine may be used as the source of chloride ions.

SUMMARY OF THE INVENTION

The present invention has been implemented in view of the foregoing situation, and has an object to provide an air filtering apparatus that does not make a user feel troublesome and can secure a chlorine concentration sufficient to perform electrolysis irrespective of a district where the apparatus is used.

Another object of the present invention is to provide an air filtering apparatus for bringing electrolytic water into contact with air in which deposition of scale can be suppressed by suppressing increase of hardness of electrolytic water.

In order to attain the above objects, according to the present invention as defined in the claims relate to an air filtering apparatus in which electrolytic water generated by an electrolytic unit is supplied to a gas-liquid contact member to infiltrate the electrolytic water into the gas-liquid contact member and reflows to a water receiving tray, air is fed to the gas-liquid contact member by an air blowing fan to bring the electrolytic water into contact with the air in the gas-liquid contact member to filter the air, wherein the apparatus comprises a brine supply unit for supplying brine to the water receiving tray; and a controller for setting a water exchange operation mode in which water stocked in the water receiving tray is discharged and new water is supplied into the water receiving tray, and operating the brine supply unit when the water exchange operation mode is selected and supplying brine into the water receiving tray.

According to this construction, brine water is automatically supplied to new water which is supplied to the water receiving tray after water is discharge.

In the air filtering apparatus according to the invention, and as otherwise defined in claim 1, the controller has an electric conductivity detecting unit for detecting the electric conductivity of water reflowing to the water receiving tray, and brine water is supplied to the water receiving tray in accordance with the electric conductivity of the water.

According to this construction, the brine is automatically supplied to the water receiving tray in accordance with the electric conductivity of water reflowing to the water receiving tray.

In the above air filtering apparatus, the new water supplied to the water receiving tray is tap water containing a low concentration of chlorine, discharge of water stocked in the water receiving tray and supply of new water are repeated until the electric conductivity of water reflowing in the water receiving tray is equal to a predetermined value or less when the water exchange operation is carried out, and the brine supply unit is operated to supply brine into the water receiving tray when the electric conductivity of the water reaches the predetermined value or less.

According to this construction, after the water stocked in the water receiving tray is exchanged by tap water containing a low concentration of chlorine, brine is automatically supplied to the water receiving tray.

Furthermore, in the above air filtering apparatus, the air filtering operation mode for filtering air is provided, and the controller stops the air filtering operation mode every predetermined time, and resumes the air filtering operation mode when the water exchange operation mode is selected.

According to this construction, water which are used while circulated is exchanged by new water every predetermined time, and condensation of electrolytic water and propagation of various kinds of bacteria, etc. are prevented. Furthermore, brine is automatically supplied to the water receiving tray every time water stocked in the water receiving tray is exchanged.

According to a second aspect not according to the present invention, an air filtering apparatus comprising: a housing that is divided into upper and lower chambers through a support plate; an electrolytic unit for generating electrolytic water; a gas-liquid contact member that is supplied with electrolytic water generated by the electrolytic unit to bring air into contact with the electrolytic water, thereby filtering the air; a water receiving tray for stocking electrolytic water generated by the electrolytic unit and receiving electrolytic water dropping from the gas-liquid contact member; a circulating pump for pumping up electrolytic water stocked in the water receiving tray and supplying the electrolytic water to the gas-liquid contact member; and an air blowing fan for blowing indoor air to the gas-liquid contact member, is **characterized in that** the electrolytic unit, the gas-liquid contact member, the water receiving tray and the circulating pump are disposed in the upper chamber of the housing while the air blowing fan is provided in the lower chamber of the housing, and a brine tank for stocking brine to be supplied to the water receiving tray is disposed in the upper chamber of the housing.

According to this construction, brine is automatically supplied to electrolytic water in the water receiving tray.

In the above air filtering apparatus, a pump for pumping up brine from the brine tank is provided, the suction port of the pump is connected to the brine tank through a one-touch joint mechanism , the discharge port of the pump is connected to a brine supply nozzle, and the brine supply noise is disposed at the upper side of the water receiving tray.

According to this construction, the brine tank can be detached from the suction port of the pump through one-touch operation, and thus the brine tank can be easily taken out. Furthermore, the supply nozzle is disposed at the upper side of the water receiving tray which is under a relatively high humid state, thereby preventing deposition of salt at the supply nozzle.

In the above air filtering apparatus, the upper chamber is partitioned into an air filtering space and a water-associated space through a partition so that the gas-liquid contact member is disposed in the air filtering space and the circulating pump, the brine tank and the supply nozzle are disposed in the water-associated space, and the pump is disposed in a humidity-low space adjacent to the water-associated space.

According to this construction, the brine tank and the supply nozzle are disposed in the water-associated space which is higher in humidity than the air filtering space. Accordingly, evaporation of water in the brine tank is made slower, brine is accommodated in the brine tank so as to keep a predetermined salt concentration, and deposition of salt at the supply nozzle can be prevented. By disposing the pump in the humidity-low space, it is unnecessary to subject the pump to a moisture-proof processing or the like.

Furthermore, in the above air filtering apparatus, a holding table on which the brine tank is mounted is provided at the upper side of the water receiving tray, and the supply nozzle for brine is mounted on the holding table.

According to this construction, it is unnecessary to separately provide a member for supporting the supply nozzle.

In the above air filtering apparatus, the supply nozzle for supplying brine to the water receiving tray is provided with a check valve, and the supply nozzle is disposed to be far away from the water level of electrolytic water in the water receiving tray.

According to this construction, electrolytic water in the water receiving tray can be prevented from flowing back into the brine tank by the check valve. Furthermore, since the supply nozzle is disposed to be far away from the water level of electrolytic water, whereby backflow of the electrolytic water can be surely prevented. Accordingly, corrosion of the inside of the check valve due to the contact between the check valve and the electrolytic water can be prevented.

In the above air filtering apparatus, the brine tank is provided with a valve device for adjusting the internal pressure of the tank.

According to this construction, the internal pressure of the brine tank can be kept constant, and the brine can be stocked at a predetermined salt concentration in the brine tank.

According to a third aspect not according to the present invention, there is provided an air filtering apparatus comprising: an electrolytic bath for electrolyzing water to generate electrolytic water containing active oxygen species; a gas-liquid contact member for bringing air into contact with electrolytic water; a circulating pump for supplying electrolytic water generated in the electrolytic bath into the gas-liquid contact member, and circulating and supplying electrolytic water passing through the gas-liquid contact member to the gas-liquid contact member again, and a water-softening module for executing a processing of reducing the hardness of electrolytic water which is circulated and supplied to the gas-liquid contact member.

According to this construction, the hardness of electrolytic to be circulated and supplied to the gas-liquid contact member is lowered by the processing of the water-softening module, and thus deposition of hardness components such as calcium, magnesium, etc. contained in electrolytic water occurs hardly in the gas-liquid contact member, etc. Accordingly, deposition of scale in the circulation passage of electrolytic water such as the gas-liquid contact member, etc. can be suppressed.

In the above construction, a stock unit for stocking electrolytic water dropping from the gas-liquid contact member may be provided, and the electrolytic water stocked in the stock unit may be supplied to the gas-liquid contact member by the circulating pump. The water-softening module may take electrolytic water stocked in the stock unit to conduct a processing for lowering the hardness of the electrolytic water, and return the processed electrolytic water to the stock unit.

In this case, the electrolytic water stocked in the stock unit is processed by the water-softening module and returned to the stock unit, and thus the hardness-lowered electrolytic water can be surely supplied to the electrolytic-water circulation passage containing the gas-liquid contact member, so that deposition of scale can be suppressed over the overall circulation passage.

The water-softening module may be disposed together with the suction pump for sucking electrolytic water of the stock unit in a water-softening unit which is constructed as a separate body from the main body of the air filtering apparatus including the electrolytic bath, the gas-liquid contact member, the circulating pump and the stock unit, and conducts a processing of lowering the hardness of the electrolytic water sucked by the suction pump. A suction pipe for connecting the stock unit of the main body of the air filtering apparatus and the suction port of the suction pump of the water-softening unit, and a return pipe for connecting the discharge port for discharging the processed electrolytic water from the water-softening module and the stock unit may be disposed.

In this case, the stock unit and the water-softening unit are connected to each other through the suction pipe and the return pipe, whereby the softening unit having the water-softening module accommodated therein can be external secured to the main body of the air filtering apparatus. Therefore, the construction that the hardness of the electrolytic water is lowered by the water-softening module to suppress deposition of scale can be easily implemented by externally securing the water-softening unit to the air filtering apparatus having no water-softening module, and the function of suppressing the deposition of scale can be added.

The electrolytic water stocked in the stock unit may be supplied to the water-softening module by the function of the circulating pump.

In this case, since the electrolytic water stocked in the stock unit is supplied to the water-softening module by the function of the circulating pump, it is unnecessary to provide a pump for supplying electrolytic water to the water-softening module. Therefore, electrolytic water can be supplied to the softening module with a simple construction, and thus deposition of scale can be suppressed.

Furthermore, a first discharge pipe connected to the stock unit and a second discharge pipe connected to a drain tank for stocking drain may be switchably connected to a discharge port for discharging the processed electrolytic water from the water-softening module.

In this case, the electrolytic water discharged from the water-softening module can be switchably discharged to any one of the stock unit and the drain tank, and the electrolytic water can be discharged to the stock unit or the drain tank in accordance with the operation condition of the air filtering apparatus. For example, when hardness components, etc are piled up in the water-softening module, the electrolytic water can be made to directly flow to the drain tank, whereby the hardness components, etc. can be discharged in a lump.

There may be provided a filter through which the processed electrolytic water discharged from the water-softening module passes.

In this case, foreign materials such as scale, hardness components, etc. contained in electrolytic water discharged from the water-softening module are caught and collected when passing through the filter, whereby the foreign materials such as the scale, the hardness components, etc. can be prevented from flowing into the circulation passage of the electrolytic water.

Furthermore, a discharge pipe for discharging processed electrolytic water from the water-softening module may be connected to a flow-in port of the electrolytic bath.

In this case, the electrolytic water softened by the water softening module is directly supplied to the electrolytic bath, and the electrolytic water to be electrolyzed in the electrolytic bath is softened and thus has a lowered hardness, so that deposition of scale in the electrolytic bath can be suppressed. Furthermore, the water softening module and the electrolytic bath are directly connected to each other, and the water-softening of the electrolytic water and the electrolysis can be performed in one pipe passage, so that the construction of the pipe can be simplified. Therefore, in the air filtering apparatus, the construction of performing the water-softening and the electrolysis can be simply implemented.

Furthermore, the water-softening module may be provided with paired electrodes disposed in the flow passage of the electrolytic water and a collecting member having electric conductivity which is electrically connected to one of the electrodes, and the hardness of the electrolytic water is lowered by applying a voltage between the electrodes.

In this case, a large quantity of hardness components contained in the electrolytic water are deposited on the collecting member which is electrically connected to one of the electrodes. Accordingly, the concentration of the hardness components in the electrolytic water can be lowered. Accordingly, at places other than the collecting member, the hardness components such as calcium, magnesium, etc. contained in electrolytic water are hardly deposited, and thus the deposition of scale in the electrolytic-water circulating passage containing the gas-liquid contact member can be suppressed.

Furthermore, the water-softening operation mode of lowering the hardness of electrolytic water and a cleaning operation mode for reversing the polarities of the electrodes at the execution time of the water-softening operation mode and applying a voltage may be executed so as to be switchable therebetween.

According to this construction, by executing the water-softening operation mode, hardness components such as calcium, magnesium, etc. contained in electrolytic water are deposited around the electrodes and the collecting member, and thus the hardness of the electrolytic water can be lowered. Furthermore, by executing the cleaning operation mode, the hardness components deposited around the electrodes and the collecting member are dissolved and exfoliated, and thus can be removed. Accordingly, the hardness of electrolytic water can be lowered while the function of lowering the hardness of the water-softening module can be kept to an excellent state at all times, and thus the deposition of scale can be suppressed.

In the cleaning operation mode, the processed electrolytic water from the water-softening module may be discharged to the drain tank.

In this case, the electrolytic water discharged in the cleaning operation mode is discharged to the drain tank, and thus the drain in the cleaning operation mode which contains lots of hardness components can be discharged to the outside of the electrolytic-water circulation passage.

According to the present invention, the brine supply unit is operated to supply brine to the water receiving tray when the water exchange operation mode is selected. Therefore, brine is automatically supplied to new water which is supplied to the water receiving tray after water is discharged. Accordingly, a user does not feel troublesome and a chlorine concentration which enables electrolysis can be secured.

Furthermore, according to the present invention, the apparatus is equipped with the water receiving tray for stocking electrolytic water generated in the electrolytic unit and receiving electrolytic water dropping from the gas-liquid contact member, the circulating pump for pumping up electrolytic water stocked in the water receiving tray and supplying the electrolytic water to the gas-liquid contact member again, and the brine tank for supplying stocked brine to the water receiving tray. Therefore, brine is automatically supplied to electrolytic water in the water receiving tray, so that a user does not feel troublesome, and the chlorine concentration which enables electrolysis irrespective of the district where the apparatus is used can be secured.

Still furthermore, according to the present invention, in the air filtering apparatus for bringing air into contact with electrolytic water, increase of the hardness of electrolytic water can be suppressed, and deposition of scale can be suppressed.

BRIEF DESCRIPTION OF THE DRAWINGS

Fig. 1 is a perspective view showing the outlook of an air filtering apparatus according to a first embodiment of the present invention;
Fig. 2 is a perspective view of the back side of the air filtering apparatus;
Fig. 3 is a perspective view showing the main construction of the inside of the air filtering apparatus;
Fig. 4 is a side cross-sectional view showing the main construction of the inside of the air filtering apparatus;
Fig. 5 is a perspective view showing a brine tank;
Fig. 6 is a longitudinally-sectional view of the air filtering apparatus which shows a peripheral portion of the brine tank;
Figs. 7A and 7B are diagrams showing an electrolytic-water supply aspect, wherein Fig. 7A is a schematic diagram showing the construction of an air filtering mechanism, and Fig. 7B is a diagram showing the details of the construction of an electrolytic bath;
Fig. 8 is a functional block diagram showing the construction of a control system of the air filtering apparatus;
Fig. 9 is a flowchart showing a brine supply operation of the air filtering apparatus;
Fig. 10 is a perspective view showing the outlook of an air filtering apparatus according to a second embodiment which is not according to the present invention;
Fig. 11 is a perspective view of the back side of the air filtering apparatus of Fig. 10;
Fig. 12 is a perspective view showing the main construction of the inside of the air filtering apparatus of Fig. 10;
Fig. 13 is a schematic diagram showing the construction of a main part for generating and circulating electrolytic water;
Fig. 14 is a schematic diagram showing a circulating passage of electrolytic water;
Fig. 15 is a schematic diagram showing a water-softening module;

Fig. 16 is a flowchart showing the operation of a cleaning operation mode;

Fig. 17 is a schematic diagram showing a circulating passage of electrolytic water according to a third embodiment which is not according to the present invention;

Fig. 18 is a flowchart showing the operation of a cleaning operation mode;

Fig. 19 is a schematic diagram showing a circulating passage of electrolytic water according to a fourth embodiment of the present invention;

Fig. 20 is a flowchart showing the operation of the cleaning operation mode;

Fig. 21 is a schematic diagram showing a circulating passage of electrolytic water according to a fifth embodiment of the present invention; and

Fig. 22 is a flowchart showing the operation of the cleaning operation mode.

## DETAILED DESCRIPTION OF THE PREFERRED EMBODIMENTS

Preferred embodiments according to the present invention will be described with reference to the accompanying drawings. First Embodiment

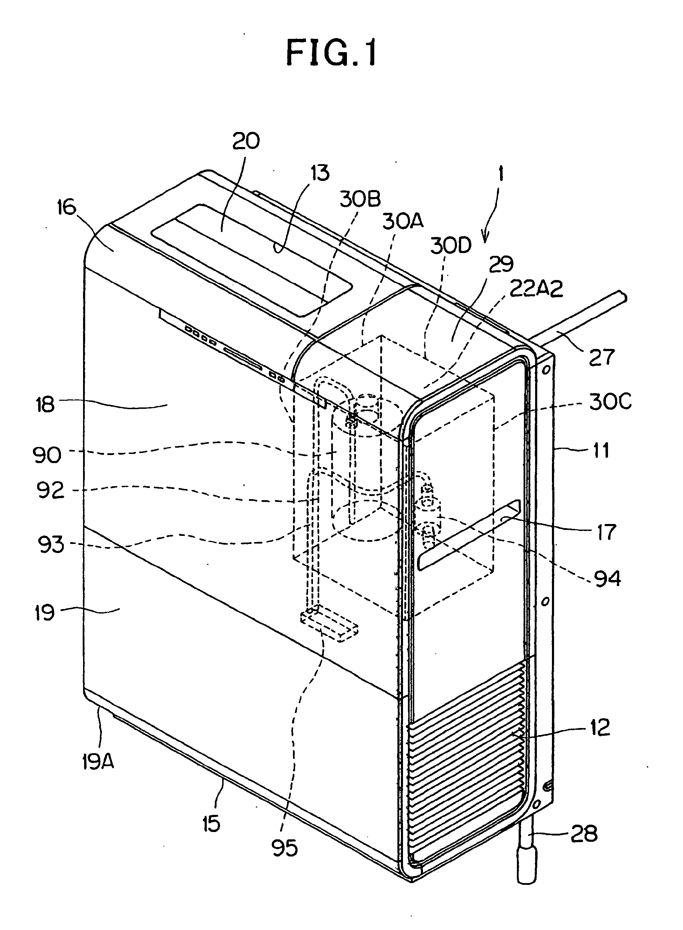

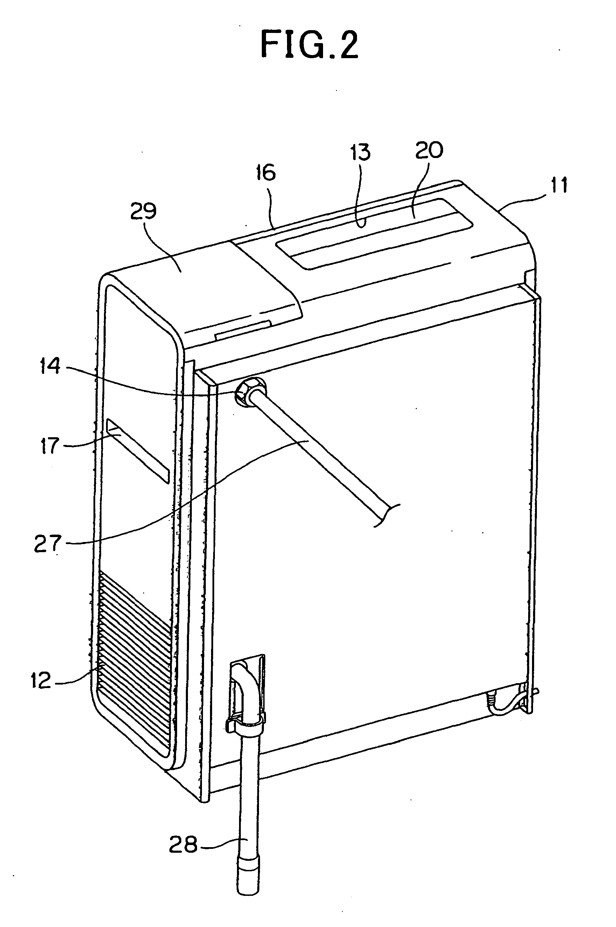

Fig. 1 is a perspective view of an air filtering apparatus 1 according to a first embodiment, and Fig. 2 is a back-side perspective view showing the air filtering apparatus 1. The air filtering apparatus 1 is an apparatus for electrolyzing water to generate electrolytic water containing predetermined active oxygen species, filtering indoor air sucked into the air filtering apparatus 1 by using the electrolytic water and blowing out the filtered clean air into a room.

As shown in Fig. 1, the air filtering apparatus 1 has a box-shaped housing 11 formed to be vertically long, and is disposed on the floor, for example. Suction grilles 12 are formed at the lower portions of both the side surfaces of the housing 11, and a suction port 15 is formed at the lower end portion of the front surface of the housing 11.

An air blow-out port 13 is formed on the top surface of the housing 11, and an automatic louver 20 for changing the air blowing direction is provided to the air blow-out port 13. The automatic louver 20 is designed to close the air blow-out port 13 when the operation is stopped.

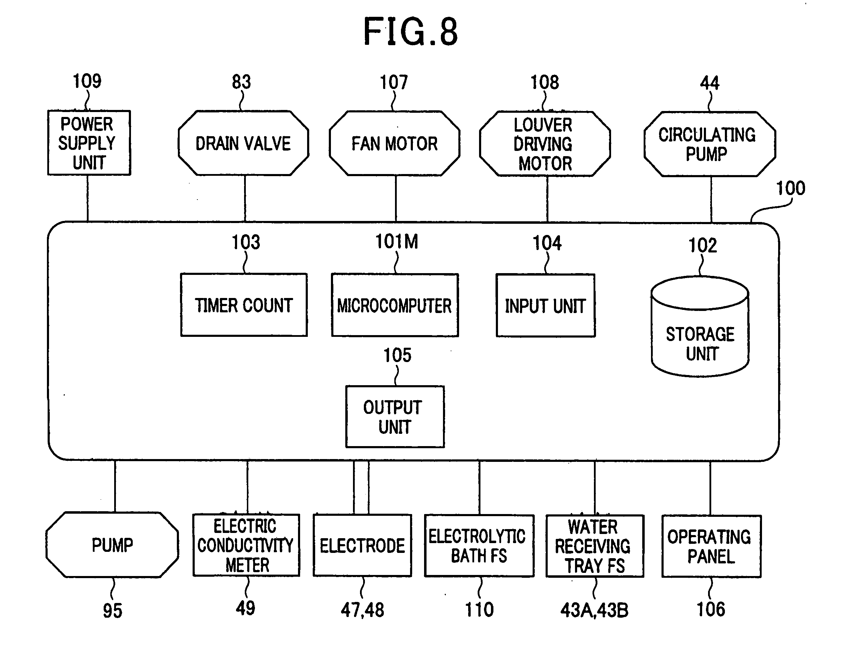

An operation lid 16 is disposed at the front side of the air blow-out port 13 on the top surface of the housing 11, and when the operation lid 16 is opened, an operating panel 106 (see Fig. 8) for performing various kinds of operations of the air filtering apparatus 1 is exposed. Furthermore, an opening/closing lid 29 for taking in and out a brine tank 90 described later is provided to one side of the top surface of the housing 11 (at the right side in front view) so as to be freely openable and closable. Grip portions 17 are formed at the upper portion of both the side surfaces of the housing 11. Each grid portion 17 is a recess portion to which a hand is hooked so that the housing 11 is carried by hands.

An upper cover member 18 and a lower cover member 19 are arranged in the vertical direction on the front surface of the housing 11 so as to be freely detachable, and the internal construction of the air filtering apparatus 1 is exposed by detaching the upper cover member 18 and the lower cover member 19. The lower cover member 19 has an arc portion 19A curved toward the back side of the housing 11 at the lower end portion of the lower cover member 19, and the suction port 15 is formed at the arc portion 19A.

As shown in Fig. 2, a connection port 14 for supplying water to the air filtering apparatus 1 is formed at the upper portion of the back surface of the housing 11, and a water supply pipe 27 connected to an external water supply source (for example, a water supply) is connected to the connection port 14. The air filtering apparatus 1 may be disposed at a district where the chlorine concentration of tap water is low, and in this case, tap water having a low chlorine concentration is supplied from the water supply pipe 27. A drain pipe 28 for discharging water in the air filtering apparatus 1 to the outside is provided at the lower portion of the back surface of the housing 11.

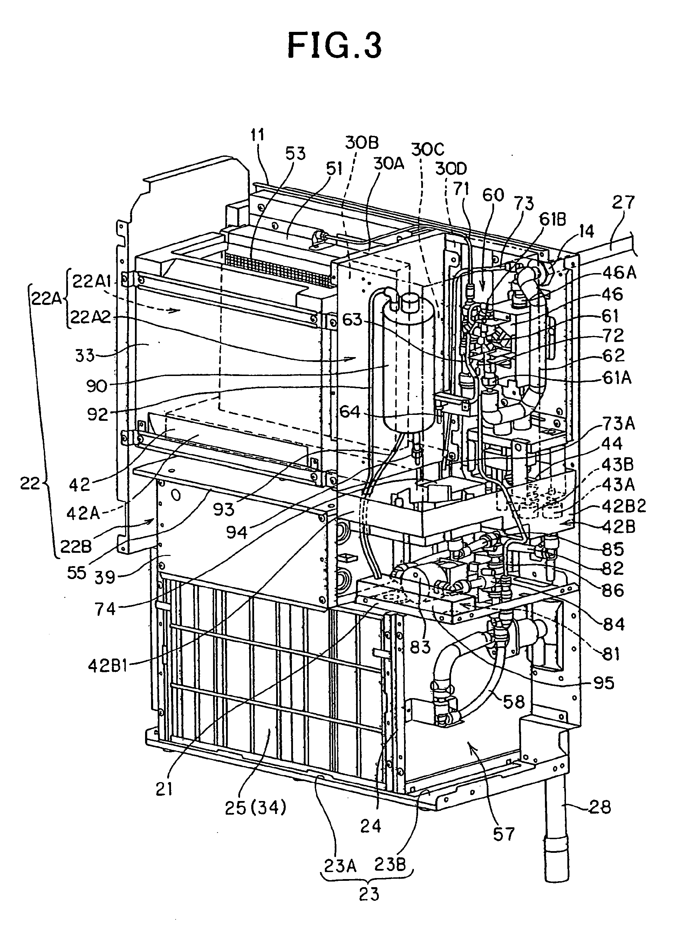

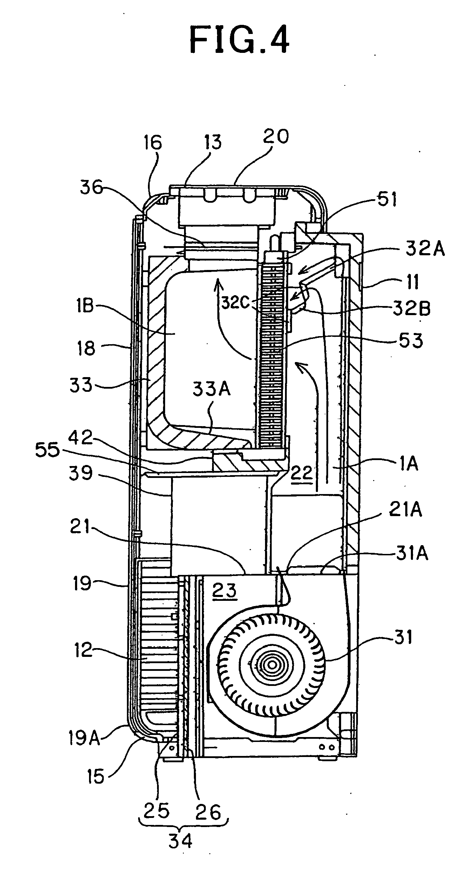

Next, the internal construction of the air filtering apparatus 1 will be described with reference to Figs. 3 and 4.

Fig. 3 is a perspective view showing the main construction of the air filtering apparatus 1, and Fig. 4 is a side cross-sectional view. As shown in Figs. 3 and 4, a support plate 21 for partitioning (compartmenting) the inside of the housing 1 into upper and lower chambers 22 and 23 is provided to the housing 11.

The lower chamber 23 is further partitioned into right and left chambers by a partition plate 24. An air blowing fan (Fig. 4) and a fan motor 107 (Fig. 8) for driving the air blowing fan 31 are accommodated in one chamber 23A, and a drain unit 57 having the drain pipe 28 is accommodated in the other chamber 23B. A pre-filter 34 is disposed at the front side of the chamber 23A so as to face the lower cover member 19 (Fig. 1). The pre-filter 34 is designed to have the size corresponding to the opening portion of the chamber 23A, and it is disposed to be fitted in the opening portion. By detaching the lower cover member 19, the pre-filter 34 is exposed, and it can be simply detached.

The pre-filter 34 comprises a rough dust filter 25 for collecting large particle-size dusts, etc. in the air sucked through the suction grilles 122 and the suction port 15, and an intermediate performance filter 26 for collecting dusts, etc. which are passed through the rough dust collector 25 and have particle diameters of $10(\mu m)$ or more (for example, grass pollen), for example. Grass pollen, dusts, etc. floating in the air sucked from the suction grilles 12 and the suction port 15 are removed by the pre-filter 34.

In the upper chamber 22, an electrical component box 39 is disposed on the support plate 21 above the chamber 23A, and a gas-liquid contact member 53 is disposed above the electrical component box 39. Furthermore, a water receiving tray 42 for receiving electrolytic water dropping from the gas-liquid contact member 53 is disposed between the electrical component box 39 and the gas-liquid contact member 53. In the electrical component box 39 are accommodated a control board on which various kinds of devices constituting a controller 100 (see Fig. 8) for controlling the air filtering apparatus 1 are mounted, and various kinds of electrical component parts such as a power supply circuit for supplying a power supply voltage to the fan motor 107, etc.

Furthermore, as shown in Fig. 4, a back-side space 1A and a front-side space 1B which are partitioned by the gas-liquid contact member 53 are formed in the upper chamber 22. The back-side space 1A intercommunicates with an air flowing port 31A of the air blowing fan 31 through an opening 21A formed in the support plate 21. Two air guide plates 32A and 32B which are inclined downwardly from the back side of the housing 11 to the front side of the housing 11 are provided at the upper portion of the back-side space 1A so as to be positionally different from each other in the height direction, and the two air guide plates 32A and 32B are supported by a frame member 32C. Therefore, air which is blown out from the air flowing port 31A of the air blowing fan 31 impinges against the two air guide plates 32A and 32B, flows along routes indicated by arrows of Fig. 4 and then are sprayed to the back surface of the gas-liquid contact member 53.

The gas-liquid contact member 53 serves to bring the air sprayed to the gas-liquid contact member 53 into contact with electrolytic water. In the gas-liquid contact member 53, air sucked into the housing 11 is brought into contact with electrolytic water containing a predetermined active oxygen species, whereby virus, etc. contained in the air are inactivated and thus the air is filtered.

A housing 33 is disposed at the front side of the gas-liquid contact member 53, and the front-side space 1B is defined by the housing 33 and the gas-liquid contact member 53. The housing 33 has a function of guiding the air in the front-side space 1B to the air blow-out port 13 and also receiving water sprayed out from the gas-liquid contact member 53 (so-called spattering water). Specifically, the housing 33 is designed so that the inner bottom surface 33A of the housing 33 is downwardly inclined to the gas-liquid contact member 53, and the tip portion of the bottom surface 33A extends to the upper side of the water receiving tray 42. Accordingly, water sprayed out to the front-side space 1B is returned to the water receiving tray 42 through the bottom surface 33A.

An air blow-out port filter 36 for preventing invasion of foreign materials from the air blow-out port 13 into the housing 11 is disposed between the housing 33 and the air blow-out port 13. It is preferable that the air blow-out filter 36 is moderately of loose texture so that the draft resistance to air passing through the gas-liquid contact member 53 is prevented from remarkably increasing.

The gas-liquid contact member 53 is a filter member having a honeycomb structure, and designed so that an element portion brought into contact with gas is supported by a frame. As not shown, the element portion is constructed by laminating a corrugated plate type corrugated member and a flat plate type flat member, and a number of substantially triangular openings are formed between the corrugated plate member and the flat plate member. Accordingly, a large gas-liquid contact area can be secured when air is passed through the element portion, and also electrolytic water can be dropped through the gas-liquid contact member, so that the gas-liquid contact member is hardly clogged.

The element portion is formed of a raw material which is little deteriorated by electrolytic water, for example, polyolefin resin (polyethylene resin, polypropylene resin, etc.), PET (polyethylene terephthalate) resin, vinyl chloride resin, fluorinated resin (PTFE, PFA, ETFE, etc.), ceramic materials, etc. It is assumed that PET resin is used in this embodiment. The element portion is subjected to a hydrophilic treatment to enhance the affinity to electrolytic water. Accordingly, electrolytic water retentivity of the gas-liquid contact member 53 (wettability) is enhanced, and the contact between active oxygen species (active oxygen material) described later and indoor air is kept for a long time.

A water sprinkling box 51 for uniformly dispersing electrolytic water onto the gas-liquid contact member 53 is installed to the upper portion of the gas-liquid contact member 53☐This water sprinkling box 51 has a tray member for temporarily stocking electrolytic water, and plural water sprinkling holes (not shown) are formed in the side surface of the tray member so that electrolytic water drops from the water sprinkling holes to the gas-liquid contact member 53.

Furthermore, a water distributing sheet (not shown) for efficiently dispersing the electrolytic water dropping from the water sprinkling box 51 to the element portion is disposed on the upper surface of the gas-liquid contact member 53. This water distributing sheet is a sheet (woven fabric, nonwoven cloth or the like) formed of a fibrous material having liquid permeability, and one or plural sheets are provided along the crosssection in the thickness direction of the gas-liquid contact member 53.

As shown in Fig. 3, the water receiving tray 42 has a water receiving unit 42A located at the lower side of the gas-liquid contact member 53, and a stock unit 42B extending to the upper side of the other chamber 23B, and the water receiving unit 42A and the stock unit 42B are formed integrally with each other. Water flowing from the water receiving unit 42A is stocked in the stock unit 42B. The stock unit 42B is constructed by a deep bottom portion 42B1 deeper than the water receiving unit 42A, and a shallow bottom portion 42B2 shallower than the deep bottom portion 42B1.

A first float switch 43A and a second flow switch 43B for detecting the water level are disposed in the deep bottom portion 42B1. The first float switch 43A operates when the water level in the stock unit 42B underruns a predetermined lower limit water level, and the second float switch 43B operates when the water level in the stock unit 42B exceeds a predetermined upper limit water level.

Furthermore, a circulating pump 44 is provided to the deep bottom portion 42B. This circulating pump 44 operates under the control of the controller 100, and a supply pipe 71 through which water stocked in the deep bottom portion 42B1 (stock unit 42B) is pumped up and supplied through the water sprinkling box 51 to the gas-liquid contact member 53 is connected to the discharge port of the circulating pump 44. The supply pipe 71 is connected to an electrolytic bath (electrolytic unit) 46 through a branch pipe 72 which is branched between the circulating pump 44 and the water sprinkling box 51.

As described later, plural electrodes are contained in the electrolytic bath 46, a voltage supplied from the controller 100 is applied between the electrodes to electrolyze water and thus generate electrolytic water. A discharge port 46A for discharging electrolytic water generated in the electrolytic bath 46 is formed on the top surface of the electrolytic bath 46, and a return pipe 73 for returning electrolytic water to the stock unit 42B is connected to the discharge port 46A.

A filter member 74 for collecting solid materials immixing water flowing into the stock unit 42B is disposed at the connection portion between the stock unit 42B and the water receiving unit 42A. The exit 73A of the return pipe 73 is provided above the filter member 74, and the solid materials (for example, scale components formed on the surfaces of the electrodes) discharged together with water from the electrolytic bath 46 can be collected. The filter member 74 is disposed at the inlet portion of the stock unit 42B under the state that it can be viewed from the upper side, and thus the exchange timing of the filter member 74 can be visually and easily determined. Furthermore, when the filter member 74 is exchanged, the filter member 74 may be detached and exchanged by fingers, and thus the maintenance can be easily performed without using any tool.

In this embodiment, a part of water pumped by the circulating pump 44 is supplied to the gas-liquid contact member 53 through the water sprinkling box 51, and the other part of water is supplied to the electrolytic bath 46. Electrolytic water generated in the electrolytic bath 46 is supplied to the stock unit 42B through the filter member 74, and electrolytic water stocked in the deep bottom portion 42B1 of the stock unit 42B is dispersively supplied to the gas-liquid contact member 53 and the electrolytic bath 46 by the circulating pump 44 again. As described above, in the electrolytic bath 46, electrolysis is repetitively executed by using electrolytic water, whereby electrolytic water containing active oxygen species of a high concentration can be generated. Furthermore, a water resource can be effectively used by using electrolytic water discharged from the gas-liquid contact member 53 while circulating the electrolytic water.

Furthermore, as shown in Fig. 3, a water supply portion 60 for supplying tap water from the water supply pipe 27 to the water receiving tray 42 is provided above the deep bottom portion 42B1. The water supply portion 60 is connected to the water supply pipe 27 through the connection port 14 formed on the back side of the housing 11. The water supply portion 60 has a water supply valve 61 which is opened/closed in accordance with the water level of the stock unit 42B, a first water supply pipe 62 connected to the connection port 14 at one end thereof and connected to the upstream-side end portion 61A of the water supply valve 61 at the other end thereof, a second water supply pipe 63 connected to the downstream-side end portion 61B of the water supply valve 61, and a water supply port 64 which is opened downwardly at the tip of the second water supply pipe 63.

The water supply valve 61 is an electromagnetic valve which is opened/closed under the control of the controller 100 in accordance with the water level detected by the first float switch 43A and the second float switch 43B. The water supply valve 61 is disposed so that the upstream-side end portion 61A is located at the lower side and the downstream-side end portion 61B is located at the upper side. That is, the water supply valve 61 is disposed so that water supplied to the water supply portion 60 flows from the lower side to the upper side in the water supply valve 61. Accordingly, when the water supply valve 61 is opened, even if water leaks from the connection portion between the upstream-side end portion 61A of the water supply valve 61 and the first water supply pipe 62, the leaking water Is not attached to the water supply valve 61. Therefore, occurrence of a trouble such as ground leakage or the like due to the water leakage can be prevented.

The water supply port 64 of the second water supply pipe 63 is disposed so as to be far away from the water level of water stocked in the water receiving tray 42 at such a sufficient distance that the water supply port 64 does not touch the water level (in this embodiment, at 35mm from the upper end surface of the water receiving tray 42). Accordingly, even when the internal pressure of the water supply portion 60 and the water supply pipe 27 is negative, water stocked in the water receiving tray 42 is prevented from flowing back into the water supply portion 60 and the water supply pipe 27 through the water supply port 64.

Furthermore, the water supply port 64 is disposed above the filter member 74. Accordingly, water supplied through the water supply port 64 drops onto the filter member 74, and thus the dropping sound can be reduced, so that silent water supply can be performed.

Still furthermore, in this embodiment, water stocked in the water receiving tray 42 can be arbitrarily discharged. Specifically, a drain valve unit 81 for discharging water stocked in the water receiving tray 42 to the drain portion 57 is disposed below the stock unit 42B. The drain valve unit 81 has a first drain pipe 82 connected to the bottom portion of the deep bottom portion 42B1 of the stock unit 42B, a drain valve 83 connected to the first drain pipe 82 and a second drain pipe 84 connected to the drain valve 83, and the second drain pipe 84 is connected to the drain portion 57. The drain valve 83 is opened/closed under the control of the controller 100.

The controller 100 may open the drain valve 83 to discharge water stocked in the deep bottom portion 42B1 through the drain portion 57 to the outside every time an accumulative operation time of an air filtering operation (normal operation) of the air filtering apparatus 1 reaches a predetermined time, every time an operation stop time of the air filtering apparatus 1 reaches a predetermined time, or every time predetermined time elapses.

An overflow pipe 85 is connected to the bottom portion of the shallow bottom portion 42B2 of the stock unit 42B, and the overflow pipe 85 is connected to the second drain pipe 84 between the drain valve 83 and the drain portion 57. Therefore, the water level in the deep bottom portion 42B1 increases, and even when this water reaches the shallow bottom portion 42B2, this water is discharged to the outside through the overflow pipe 85, the second drain pipe 84 and the drain portion 57.

Furthermore, An air-bleeding pipe 86 having a smaller diameter than the second drain pipe 84 is connected to the second drain pipe 84. This air-bleeding pipe 86 is used to discharge air in the drain valve unit 81 to the outside at the drainage time, and the tip of the air-bleeding pipe 86 is disposed at a sufficiently higher position than the water receiving tray 42.

The drain portion 57 has a trap pipe 58 connected to the second drain pipe 84, and a drain pipe 28 connected to the trap pipe 58. The trap pipe 58 is designed so that water is trapped in the trap pipe 58. Therefore, the drain pipe 28 and the drain valve unit 81 are isolated from each other by water trapped in the trap pipe 58, so that smell of drain is prevented from spreading in the air filtering apparatus 1.

As shown in Fig. 3, the air filtering apparatus of this embodiment has a freely detachable brine tank (brine supplying unit) 90 for supplying brine to the water receiving tray 42.

The upper chamber 22 is further partitioned into upper and lower parts by the water receiving tray 42 and a planar member 55, and has a high-humidity space 22A which is located above the water receiving tray 42 and relatively high in humidity, and a low-humidity space 22B which is located below the water receiving tray 42 and relatively low in humidity. The electrical component box 39 described above is disposed in the low-humidity space 22B. A partition wall 30A through which the high-humidity space 22A is partitioned into right and left parts is provided in the high-humidity space 22A, and the high-humidity space 22A is partitioned into an air filtering space 22A1 in which the gas-liquid contact member 53 is accommodated, and a water-associated space 22A2 in which the circulating pump 44 is accommodated. The water-associated space 22A2 is isolated by the water receiving tray 42, the partition wall 30A and water-associated covers 30B to 30D covering the side surface of the water-associated space 22A2, and the water-associated space 22A2 is higher in humidity than the air filtering space 22A1 in which air flows.

The brine tank 90 for stocking brine is disposed in the high-humidity water-associated space 22A2. Accordingly, vaporization of water in the brine tank 90 is made slower, and thus brine in the tank can be stocked at a predetermined chlorine concentration.

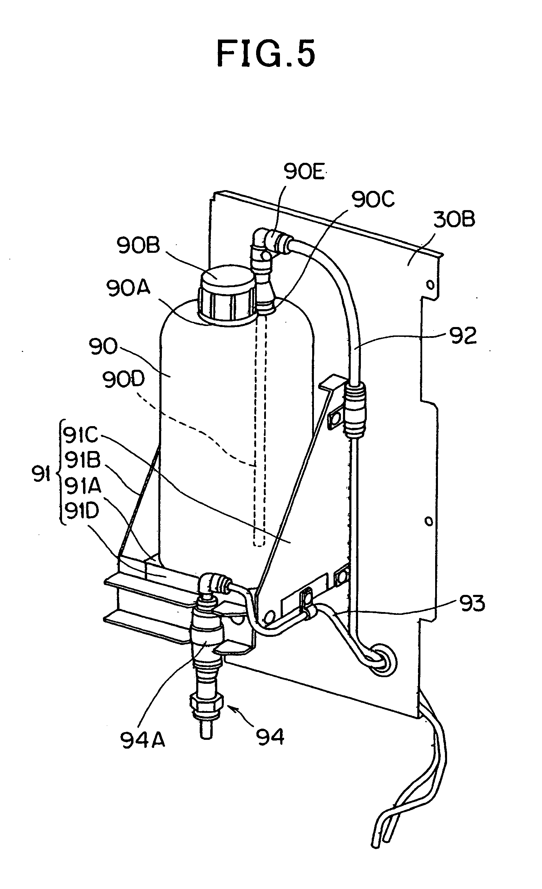

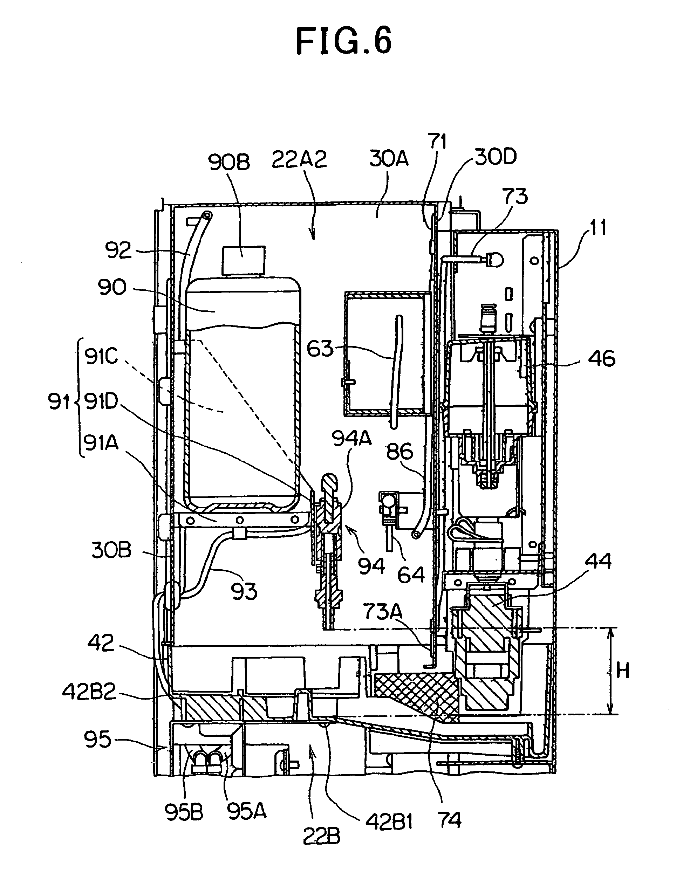

Fig. 5 is a perspective view showing the brine tank 90. Fig. 6 is a longitudinally sectional view of the air filtering apparatus 1 which shows the peripheral portion of the brine tank 90.

The brine tank 90 is formed of a material which is little deteriorated by brine, for example, polypropylene resin or the like. The brine tank 90 is designed to be so large that a sufficient amount of brine can be stocked so as to be supplied for a long term with respect to a maintenance period (for example, one year) of the air filtering apparatus 1. When the volume of the brine tank 90 of this embodiment is set to 1 liter, the maintenance period of the brine tank 90 is equal to two years.

It is preferable that the salinity (concentration of salt) (wt%) of brine stocked in the brine tank 90 is equal to 20% or more to suppress breeding of fungus and also equal to or less than 26% (saturation concentration) at which deposition of salt starts. In this embodiment, salt having sodium chloride of 99% or more in purity is used to obtain brine whose salinity is adjusted to 20%, and the brine thus generated is stocked in the brine tank 90.

As shown in Fig. 5, a water supply port 90A for supplying brine to the brine tank 90 is provided to the upper surface of the brine tank 90. A valve device 90B for adjusting the internal pressure in the brine tank 90 is secured to the water supply port 90A. The valve device 90B can keep the pressure in the brine tank 90 to a constant state, so that the brine can be accommodated at a predetermined salinity in the brine tank 90.

A suction port 90C is provided to the upper surface of the brine tank 90, and a suction nozzle 90D extends from the suction port 90C into the brine tank 90. A one-touch joint member 90E is secured to the suction port 90C.

The brine tank 90 is mounted on a holding table 91 disposed above the water receiving tray 42 (see Fig. 6). The holding table 91 is fixed to a water-associated cover 30B disposed on the front surface of the air filtering apparatus 1. Accordingly, the brine tank 90 is disposed at the front side of the air filtering apparatus 1, and thus the brine tank 90 can be easily taken into/out from the opening/closing lid 29 (see fig. 1).

The holding table 91 has a rectangular bottom surface 91A which is horizontally fixed to the water-associated cover 30B, a side surface 91B which erects from the bottom surface 91A and is vertically fixed to the water-associated cover 30B, and a side surface 91D which erects from the bottom surface 91A in parallel to the water-associated cover 30B. When the brine tank 90 is disposed on the bottom surface 91A, it is surrounded by the side surfaces 91B to 91d. Therefore, even when the air filtering apparatus 1 is swung in some degree during transport, it is prevented from falling off the holding table 91.

The brine tank 90 is merely mounted on the bottom surface 91A without being fixed to the holding table 91 by bolting or the like, and a polyurethane tube 92 is connected to the one-touch joint member 90E. When the brine tank 90 is taken out, this tube 92 can be easily detached from the one-touch joint member 90E by fingers, and thus the maintenance can be easily performed without using any tool or the like.

The tube 92 is joined to the tube 93, and the tube 93 is connected to the supply nozzle 94 secured to the bottom surface 91A of the holding table 91. As described above, the supply nozzle 94 is supported on the holding table 91 on which the brine tank 90 is mounted, whereby it is unnecessary to separately provide a member for supporting the supply nozzle 94 and thus the cost-up can be suppressed.

The supply nozzle 94 has a check valve 94A for preventing backflow. As shown in Fig. 6, the supply nozzle 94 is disposed so as to be far away from the water level of water stocked in the water receiving tray 42 at such a sufficient distance that it does touch the water level (in this embodiment, the distance H from the bottom surface of the water receiving tray 42 is set to 62mm). Accordingly, even when the internal pressure of the tubes 92, 93 and the brine tank 90 is negative, electrolytic water stocked in the water receiving tray 42 can be surely prevented from flowing back through the supply nozzle 94 into the tubes 92, 93 and the brine tank 90. Therefore, it is unnecessary to form the inside of the check valve 94A of an expensive material which is hardly deteriorated by electrolytic water, and thus the cost-up of the check valve 94A can be suppressed.

The supply nozzle 94 is disposed in the high-humidity water-associated space 22A2 to be located above the electrolytic water stocked in the water receiving tray 42, so that it is placed under a high-humidity state at all times. Therefore, deposition of salt in the supply nozzle 94 is prevented.

The supply nozzle 94 is disposed above the deep bottom portion 42B1 (stock unit 42B) which is deeper than the shallow bottom portion 42B2 of the water receiving tray 42. Therefore, even when the water level of the stock unit 42B of the water receiving tray 42 reaches a predetermined lower limit water level, brine is directly supplied to the electrolytic water in the stock unit 42B, and thus deposition of salt on the water receiving tray 42 is prevented.

The brine stocked in the brine tank 90 is pumped up by a pump 95 operated under the control of the controller 100. A tube 92 joined to the brine tank 90 is connected to the suction port 95A of the pump 95. Furthermore, a tube 93 joined to the supply nozzle 94 is connected to the discharge port 95B of the pump 95. These tubes 92 and 93 penetrate from the water-associated space 22A2 through the water-associated cover 30B and extend into the low-humidity space 22B adjacent to the water-associated space 22A2. The pump 95 is disposed in the low-humidity space 22B. As described above, the pump 95 is disposed at the low-humidity space 22B which is relatively low in humidity, and thus a pup which is not subjected to a damp proof treatment or the like may be used as the pump 95.

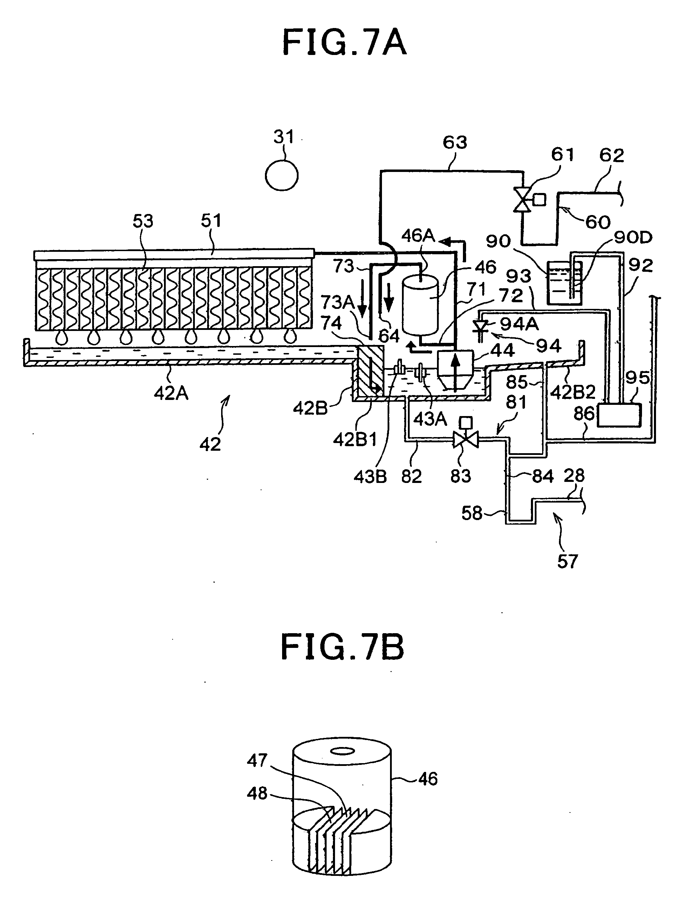

Fig. 7A is a schematic diagram showing the construction of the air filtering mechanism and supply flow of electrolytic water, and Fig. 7B shows the detailed construction of the electrolytic bath 46. The supply of electrolytic water to the gas-liquid contact member 53 will be described with reference to Figs. 7A and 7B.

When the air filtering apparatus 1 is operated, the water level of the stock unit 42B is detected, and when this water level does not reach a predetermined level, the water supply valve 61 is opened to supply tap water to the water receiving tray 42, whereby the water level of the stock unit 42B of the water receiving tray 42 reaches a predetermined water level.

Water in the stock unit 42B is pumped up by the circulating pump 44, and a part thereof is supplied to the electrolytic bath 46. As shown in Fig. 7B, the electrolytic bath 46 has a pair of electrodes 47 and 48, and one of the electrodes 47 and 48 serves as an anode while the other electrode serves as a cathode. By applying a voltage between the electrodes 47 and 48, tap water flowing into the electrolytic bath 46 is electrolyzed to generate electrolytic water containing active oxygen species. Here, the active oxygen species means oxygen having higher oxidation activity than normal oxygen and relevant materials thereto. For example, it contains so-called narrowly-defined active oxygen such as superoxide anion, singlet oxygen, hydroxyl radical, hydrogen peroxide, etc. and so-called broadly-defined active oxygen such as ozone, hypohalous acid, etc.

Each of the electrodes 47 and 48 is an electrode plate comprising a base of titan (Ti) and a coating layer of iridium (Ir), platinum (Pt).

When a voltage is applied from an external power source between the electrode 47 serving as an anode and the electrode 48 serving as a cathode, hydrogen ion ($H^+$) and hydroxide ion ($OH^-$) in water react with each other at the electrode 48 serving as the cathode according to the following reaction formula (1).

$$4H^+ + 4e^- + (4OH^-) \rightarrow 2H_2 + (4OH^-) \qquad (1)$$

On the other hand, at the electrode 47 serving as the anode (positive electrode), water is electrolyzed according to the following formula (2).

$$2H_2O \rightarrow 4H^+ + O_2 + 4e^- \qquad (2)$$

In addition, at the electrode 47, chlorine ions (chloride ion: Cl⁻) contained in water reacts according to the following formula (3), and chlorine ($Cl_2$) is generated.

$$2Cl^- \rightarrow Cl_2 + 2e^- \qquad (3)$$

Furthermore, this chlorine reacts with water according to the following formula (4), and hypochlorous acid (HClO) and hydrogen chloride (HCl) occur.

$$Cl_2 + H_2O \rightarrow HClO + HCl \qquad (4)$$

Hypochlorous acid occurring at the electrode 47 is contained in the broadly-defined active oxygen species, and has strong oxygen action and bleaching action. Water in which hypochlorous acid is dissolved, that is, electrolytic water generated in the air filtering apparatus have various kinds of air cleaning effects such as inactivation of virus, etc., sterilization, decomposition of organic compounds, etc.

When hypochlorous acid having strong sterilization power is generated by the electrodes 47, 48, electrolytic water containing hypochlorous acid is dropped from the water sprinkling box 51 to the gas-liquid contact member 53, air blown out from the air blowing fan 31 comes into contact with hypochlorous acid in ht gas-liquid contact member 53. Accordingly, virus, etc. floating in the air are inactivated, and odor material contained in the air concerned reacts with hypochlorous acid to be decomposed or ionized and thus dissolved. Accordingly, air filtering and deodorization are performed, and cleaned air is discharged from the gas-liquid contact member 53.

An inactivating mechanism of virus, etc. by the active oxygen species will be described by exemplifying influenza virus. The active oxygen species functions to break down and vanish (remove) the surface protein (spike) of the virus concerned which is indispensable for infection. When the surface protein of influenza virus is broken down, the influenza virus is not joined to a receptor which is necessary for infection of the virus concerned, so that infection can be prevented. Therefore, influenza virus floating in the air is brought into contact with the electrolytic water containing the active oxygen species in the gas-liquid contact member 53, so that the influenza virus loses so-called infection power, and thus the infection can be prevented.

Accordingly, even when the air filtering apparatus 1 is placed in a so-called large space such as a kindergarten, an elementary/junior high/high school, long-term care insurance facilities, a hospital or the like, air which is cleaned (filtered, deodorized, etc.) by electrolytic water can be broadly spread in the large space, and thus the air filtering and deodorizing operation can be efficiently performed in the large space.

The concentration of the active oxygen species in electrolytic water is adjusted so that virus, etc. to be filtered can be inactivated. The adjustment of the concentration of the active oxygen species is performed by adjusting the voltage to be applied between the electrodes 47 and 48 to adjust the value of current flowing between the electrodes 47 and 48. In this case, the adjustment may be performed in consideration of a current supply time, too. The electrolysis condition of the voltage, the current, the current supply time, etc. is preset in the air filtering apparatus 1.

For example, when positive potential is applied to the electrode 47 so that the current value flowing between the electrodes 47 and 48 is equal to 20mA (milliamperes)/cm² (square centimeter) in current density, a predetermined free residual chlorine concentration (for example, 1mg (milligrams)/l (liter)) is generated. By changing the voltage applied between the electrodes 47 and 48 to increase the current value, the concentration of hypochlorous acid in electrolytic water can be increased.

Electrolytic water dropping from the water sprinkling box 51 to the gas-liquid contact member 53 moves downwardly along the gas-liquid contact member 53, and drops onto the water receiving unit 42A of the water receiving tray 42. The electrolytic water dropping onto the water receiving unit 42A flows into the stock unit 42B through the filter member 74. Then, the electrolytic water is pump up by the circulating pump 44 again, passed through the electrolytic bath 46 and then supplied to the gas-liquid contact member 53. As described above, a circulation system in which water reflows to the water receiving tray 42 is adopted in the construction of this embodiment, and a small amount of water can be effectively used. Accordingly, air filtering operation can be efficiently performed for a long term. When the water level of the stock unit 42B is reduced due to vaporization or the like, the water supply valve 61 is opened and a proper amount of tap water is supplied from the water supply port 64.

Furthermore, when the pup 95 is driven under the control of the controller 100, brine stocked in the brine tank 90 is sucked out from the suction nozzle 90D, passed through the tubes 92 and 93, and then automatically supplied from the water supply nozzle 94 to the water receiving tray 42. Accordingly, even when tap water having a low chlorine concentration is supplied to the water receiving tray 42, chloride ion is contained in water supplied to the electrolytic bath 46.

Furthermore, electrolytic water which is circulated and used is exchanged. Therefore, electrolytic water stocked in the water receiving tray 42 is discharged every predetermined time, and a proper amount of tap water may be supplied to the water receiving tray 42. In this case, when electrolytic water in the water receiving tray 42 is exchanged, the pump 95 is operated and brine stocked in the brine tank 90 is sucked from the suction nozzle 90D, passed through the tubes 92 and 93 and then automatically supplied from the supply nozzle 94 into the water receiving tray 42. The brine is supplied to the water receiving tray 42 until the salinity (the concentration of chlorine) of water supplied to the electrolytic bath 46 reaches a predetermined value. The chlorine concentration is determined by measuring the electric conductivity of water supplied to the electrolytic bath 46.

When brine is supplied, the pump 95 is controlled by the controller so that the amount of one drop of brine is equal to 1 to 2ml. The supply nozzle 94 is provided with a check valve 94A, and brine is sharply dropped, and a proper amount of brine which corresponds to the chlorine concentration of water supplied to the electrolytic bath 46 can be supplied.

Water supplied to the electrolytic bath 46 is supplied with brine containing chlorine (chloride) ion, and thus the chlorine (chloride) ion is increased. Accordingly, when the water is electrolyzed, this chlorine (chloride) ion reacts according to the formulas (3) and (4), and hypochlorous acid and hydrochloric acid are generated. Accordingly, even when the air filtering apparatus 1 uses tap water having a low chlorine concentration or is used in a district where the chlorine concentration of tap water is low, electrolytic water containing active oxygen species is stably generated and exercises a sufficient air filtering effect (inactivation, sterilization, deodorization, etc. of virus, etc.).

In the air filtering apparatus 1 of this embodiment, by increasing the chlorine ion of water to be electrolyzed, the current to flow between the electrodes 47 and 48 under electrolysis can be reduced. Accordingly, active oxygen species can be efficiently generated, and also the load imposed on the electrodes 47 and 48 is reduced, so that the exchange frequency of the electrodes 47 and 48 can be reduced.

As described above, according to this embodiment, the air filtering apparatus 1 in which water is circulated is provided with the brine tank 90 for supplying stocked brine to the water receiving tray 42, whereby electrolytic water in the water receiving tray 42 is automatically supplied with brine. Accordingly, the user does not feel troublesome and the chlorine concentration which enables electrolysis at all times can be secured irrespective of the district where the apparatus is used.

Furthermore, according to this embodiment, the brine tank 90 is not fixed, but merely mounted on the holding table 91, and connected to the suction port 95A of the pump 95 through the one-touch joint member 90E and the tube 92. Therefore, the brine tank 90 can be removed from the tube 92 through one-touch operation, and the brine tank 90 can be easily removed. Furthermore, the supply nozzle 94 is disposed above the water receiving tray 42 which is relatively high in humidity, whereby deposition of salt at the supply nozzle 94 can be prevented.

Furthermore, according to this embodiment, the brine tank 90 and the supply nozzle 94 are disposed in the water-associated space 22A2 which is higher in humidity than the air filtering space, whereby vaporization of water in the brine tank 90 is made slower and a predetermined salt concentration of brine can be accommodated in the tank. In addition, deposition of salt at the supply nozzle 94 can be prevented. Furthermore, by disposing the pump 95 in the low-humidity space 22B, it is unnecessary to execute the damp proofing treatment on the pump 95, and thus the cost-up of the pump 95 can be suppressed. Furthermore, the degree of freedom of the pump can be increased.

According to this embodiment, the supply nozzle 94 is provided to the holding table 91 on which the brine tank 90 is mounted, so that it is unnecessary to separately provide a member for supporting the supply nozzle 94 and thus the cost-up can be suppressed.

Furthermore, according to this embodiment, the check valve 94A is provided to the supply nozzle 94 for supplying brine to the water receiving tray 42, whereby electrolytic water of the water receiving tray 42 can be prevented from flowing back into the brine tank 90. In addition, the supply nozzle 94 is disposed far away from the water level, whereby the electrolytic water can be surely prevented from flowing back. Furthermore, the corrosion of the inside of the check valve 94A due to the contact between the check valve 94A and electrolytic water can be prevented.

Still furthermore, according to this embodiment, the brine tank 90 is provided with the valve device 90B for adjusting ht internal pressure of the tank, whereby the pressure in the brine tank 90 can be kept to a constant state, and brine can be accommodated at a predetermined salt concentration (salinity).

Next, the control system of this embodiment will be described in detail.

Fig. 8 is a functional block diagram showing the construction of the control system of the air filtering apparatus 1. As shown in Fig. 8, not only the circulating pump 44, the drain valve 83, the pump 95 and the fan motor 107, but also a louver driving motor 108 for opening/closing the automatic louver 20 and a power supply unit 109 for supplying power to the respective parts described above are connected to the controller 100, and they are operated under the control of the controller 100. Various kinds of switches, an indicator lamp, etc. disposed on the operating panel 106 are connected to the controller 100, and the first flow switch (FS) 43A and the second float switch (FS)43B of the water receiving tray 42, the electrodes 47 and 48, the electrolytic bath float switch (FS) 110 for detecting the water level in the electrolytic bath 46 and the electric conductivity meter (electric conductivity detecting unit) 49 for detecting the electric conductivity between the electrodes 47 and 48.

The electric conductivity meter 49 is disposed in the electrolytic bath 46 so as to detect the electric conductivity of electrolytic water in the electrolytic bath 46, however, it is not limited to this style. It may be disposed so as to measure the electric conductivity of water circulating through the water receiving tray 42.

The controller 100 has a microcomputer 101M for controlling the overall air filtering apparatus 1, a storage unit 102 for storing control programs to be executed in the microcomputer 101M, data such as control parameters, etc., a timer counter 103 for executing a time counting operation under the control of the microcomputer 101M, an input unit 106 for detecting the operation of the operating panel 106 and outputting an operation content to the microcomputer 101M, and an output unit 105 for outputting a processing result of the microcomputer 101M by controlling turn-on of an indicator lamp (not shown) of the operating panel 106 or the like.

The microcomputer 101M reads and executes a control program which is stored in the storage unit 102 in advance, and also reads control parameters stored in the storage unit 102 to operate the respective parts of the air filtering apparatus 1. The microcomputer 101M of this embodiment selects an air filtering operation mode for executing an air filtering operation of filtering air, a water exchange operation mode for exchanging water stocked in the water receiving tray 42 and supplying brine, and a maintenance operation mode for draining electrolytic water.

The operation mode is normally set to the air filtering operation mode. When the air filtering operation mode is selected, the microcomputer 101M is instructed to start the operation on the operating panel 106, and when information representing this operation is input from the input portion 104, the circulating pump 44 is operated to start water circulation, and also a voltage is applied between the electrodes 47 and 48 to generate electrolytic water. Furthermore, the microcomputer 101M operates the louver driving motor 108 to sets the automatic louver 20 is set to the open state, and then the operation of the fan motor 107 is started so that the air blowing operation of the air blowing fan 31 is started. Through the above series of operations, the air filtering operation of the air filtering apparatus 1 is started. In connection with the start of the air filtering operation, the microcomputer 101M instructs the output unit 105 to make a display that the apparatus is under operation.

Furthermore, the microcomputer 101M instructs the timer counter 103 to start the counting of the operation time in connection with the start of the air filtering operation. The timer counter 103 can accumulatively count the operation time, and even when the air filtering apparatus 1 stops the air filtering operation, the timer count 103 counts the operation time subsequently to the previously counted operation time when the air filtering operation is resumed. The timer counter 103 notifies the count value to the microcomputer 101M and resets the count value every time the count value reaches a predetermined count value (accumulated operation time).

During execution of the air filtering operation, when it is detected by the electrolytic bath float switch 110 that the water level in the electrolytic bath 46 decreases to a low water level and also when it is detected by the first float switch 43A of the water receiving tray 42 that the water level of the water receiving tray 42 decreases to a low water level, the microcomputer 101M stops to apply the voltage to the electrodes 47, 48, stops the operation of the circulating pump 44 and the fan motor 107, and also instructs the output unit 105 to make an alarm display.

When an instruction of stopping the operation is operated in the operating panel 106 and information indicating this operation is input from the input unit 104, the microcomputer 101M stops the application of the voltage to the electrodes 47, 48, and stops the circulating pump 44. Furthermore, the microcomputer 101M stops the fan motor 107, stops the air blowing operation of the air blowing fan 31 and then operates the louver driving motor 108 to set the automatic louver 20 to a close state. Through the series of operations described above, the air filtering operation of the air filtering apparatus 1 is stopped. When the air filtering operation is stopped, the microcomputer 101M instructs the output unit 105 to stop the display indicating that the operation is being carried out, and also instructs the timer counter 103 to stop the counting of the operation time.

Furthermore, when the accumulated operation time of the air filtering operation reaches a predetermined time (for example, 40 hours), that is, when the count value of the timer counter 103 reaches a preset value, the microcomputer 101M changes the air filtering operation mode to the water exchange operation mode. During this water exchange operation mode, the microcomputer 101M executes the water exchange operation of exchanging electrolytic water which has been circulated and used.

In the water exchange operation, the microcomputer 101M opens the drain valve 83 to discharge water in the water receiving tray 42 to the outside. As described above, the first float switch 43A of the water receiving tray 42 is switched to ON when the water level of the stock unit 42B underruns the predetermined lower limit water level. Therefore, when the first float switch 43A is set to ON, the water level of the water receiving tray 42 is lower than the predetermined level, and most of water stocked in the water receiving tray 42 is discharged. Therefore, most of water existing in the electrolytic water circulation passage containing the water receiving tray 42, the circulating pump 44, the electrolytic bath 46, the water sprinkling box 51 and the gas-liquid contact member 53, that is, most of the electrolytic water used during the air filtering operation is discharged. When the first float switch 43A of the water receiving tray 42 is switched to ON, the microcomputer 101M closes the drain valve 83 and completes the drainage.

Subsequently, the microcomputer 101M opens the water supply valve 61 to start supply of water from the water supply portion 60 to the water receiving tray 42. As described above, the second float switch 43B of the water receiving tray 42 is switched to ON when the water level of the stock unit 42B exceeds the predetermined upper limit water level. When the second float switch 43B is switched to ON, the microcomputer 101M closes the water supply valve 61 and completes the water supply because sufficient water required for the air filtering operation is stocked in the water receiving tray 42. Through the above processing, the water exchange operation of exchanging water stocked in the water receiving tray 42 is completed.

Furthermore, according to this embodiment, during this water exchange operation mode, brine is supplied to the water receiving tray 42 to increase the chlorine concentration of water to be supplied to the electrolytic bath 46. The water exchange operation and the brine supply operation of supplying brine will be described hereunder.

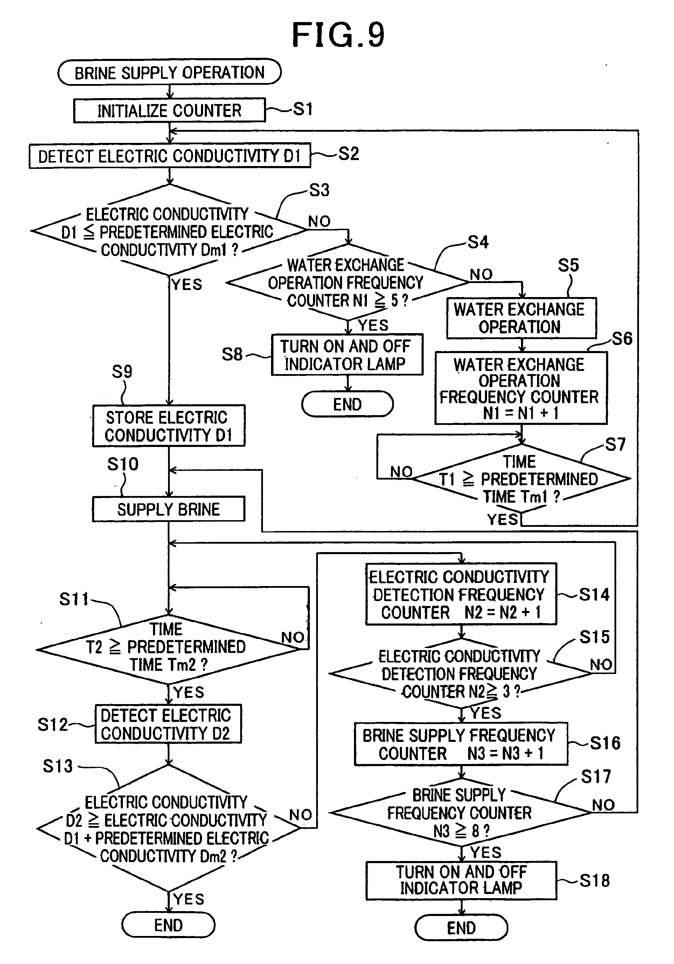

Fig. 9 is a flowchart showing the brine supply operation of the air filtering apparatus 1. During the brine supply operation shown in Fig. 9, the microcomputer 101M implements the function as a controller, and the time counter 103 implements the function as a time counting unit.

When the accumulated operation time of the air filtering operation reaches a predetermined time, the microcomputer 101M stops the air filtering operation and resets the count value of the timer counter 103, and also initializes a count value N1 of a water exchange operation frequency counter described later, a count value N2 of an electric conductivity detection frequency counter and a count value N3 of a brine supply frequency counter to "0" (step S1).

Subsequently, the microcomputer 101M instructs the electric conductivity meter 49 to detect the electric conductivity D1 of water circulated through the water receiving tray 42 (step S2), and determines whether the electric conductivity D1 is equal to a preset electric conductivity Dm1 or less (step S3). Here, the electric conductivity of water circulated in the water receiving tray 42 is detected to estimate the concentration of ion species in the water. For example, when the accumulated operation time of the air filtering operation exceeds 40 hours, the electric conductivity of electrolytic water which is circulated and used is normally equal to a high value of 6000 to 8000 $\mu$S/cm. When the electric conductivity of water supplied to the electrolytic bath 46 is excessively high, the rate of ions (for example, sulfuric acid ion, calcium ion, etc.) other than chloride ion is high, and thus it is impossible to generate electrolytic water containing hypochlorous acid whose concentration is enough to inactivate virus, etc. Accordingly, the upper limit value the predetermined electric conductivity Dm1 of this embodiment to efficiently generate electrolytic water containing hypochlorous acid whose concentration is enough to inactivate virus, etc. is set to 2000 $\mu$S (Siemens)/cm.

When the electric conductivity D1 is larger than the predetermined electric conductivity Dm1 (step S3: N), the microcomputer 101M determines whether the count value N1 of the water exchange operation frequency counter is equal to five times or more (step S4). When the count value N1 of the water exchange operation frequency counter is smaller than five times (step S4: N), the microcomputer 101M executes the water exchange operation described above to exchange circulated and used electrolytic water (step S5), and the count value N1 of the water exchange operation frequency counter is incremented by only "1" (step S6).

When the water exchange operation is finished, the microcomputer 101M starts the operation of the circulating pump 44 to circulate new water supplied to the water receiving tray 42 into the electrolytic water circulation passage, and determines whether a time T1 elapsing since the end of the water exchange operation is equal to a predetermined preset time Tm1 or more (step S7). This predetermined time Tm1 may be set to such a sufficient time that new water supplied to the water receiving tray 42 flows into the electrolytic bath 46, and the predetermined time Tm1 of this embodiment is set to 5 minutes.

The microcomputer 101M returns the processing flow to step 2 to detect the electric conductivity D1 again.

When the count value N1 of the water exchange operation frequency counter is equal to five or more as a result of the repetitive execution of the water exchange operation (step S4: Y), the microcomputer 101M determines that the water exchange operation is defective, blinks the indicator lamp and stops the operation of the air filtering apparatus 1 (step S8).

When the electric conductivity D1 is not more than the predetermined electric conductivity Dm1 (step S3: Y), the microcomputer 101M determines whether water is sufficiently exchanged, and the electric conductivity D1 is stored in the storage unit 102 (step S9). In general, the electric conductivity of tap water is equal to several hundreds micro Siemens (for example, 100 to 200 $\mu$S/cm), and thus electrolytic water containing hypochlorous acid whose concentration is enough to inactivate virus, etc. can be formed from this tap water. On the other hand, in the air filtering apparatus of this embodiment, tap water whose chlorine concentration is too low to generate electrolysis. Therefore, the microcomputer 101M drives the pup 95 and supplies a proper amount brine from the brine tank 90 into the water receiving tray 42 in order to increase the chloride ion of water to be supplied to the electrolytic bath 46 (step S10). This pump 95 is operated for one to two seconds so that the drop amount of brine is equal to 1 to 2ml. As described above, the supply nozzle 94 is provided with the check valve 94A. Therefore, brine can be sharply dropped, and a desired amount of brine can be surely supplied to the water receiving tray 42.

Subsequently, the microcomputer 101M determines whether the time T2 elapsing from the supply of brine is not less than a predetermined preset time Tm2 (step S11). This predetermined time Tm2 may be set to such a sufficient time that brine supplied to the water receiving tray 42 flows into the electrolytic bath 46, and the predetermined time Tm2 of this embodiment is set to 3 minutes.

When the predetermined time Tm2 elapses from the supply of brine (step S11: Y), the microcomputer 101M instructs the electric conductivity meter 49 to detect the electric conductivity D2 of water reflowing (circulated) to the water receiving tray 42 (step S12), and determines whether the electric conductivity D2 after brine is supplied is larger than the electric conductivity D1 stored in the storage unit 102 by a predetermined electric conductivity Dm2 or more (step S13). As described above, the difference between the electric conductivity D1 before brine is supplied and the electric conductivity D2 after brine is supplied is determined on the basis of the predetermined electric conductivity Dm2, whereby the concentration of chlorine ion which is increased by the supply of brine can be surely estimated.

At this time, when the electric conductivity of water to be supplied to the electrolytic bath 46 is excessively low, chlorine ion serving as a reference material when hypochlorous acid is generates is short, and it is impossible to generate electrolytic water containing hypochlorous acid whose concentration is enough to inactivate virus, etc. Therefore, the predetermined electric conductivity Dm2 is set so that electrolytic water containing hypochlorous acid whose concentration is enough to inactivate virus, etc. can be generated over a predetermined accumulated operation time. Furthermore, this predetermined electric conductivity Dm2 is set to be substantially equal to a conventional electrolysis condition under which a predetermined amount (for example, 0.5g) of salt is supplied to tap water. Accordingly, the conventional electrolysis condition can be used, and thus the electrolysis condition can be easily set. Still furthermore, current to flow between the electrodes 47 and 48 for electrolysis is set to be substantially equal to the conventional value Therefore, even when tap water of a low chlorine concentration is used, the load imposed on the electrodes 47, 48 is not increased, and the exchange frequency of the electrodes 47 and 48 is not required to be increased. The predetermined electric conductivity Dm2 may be set so as to satisfy the setting condition as described above, and the predetermined electric conductivity Dm2 of this embodiment is set to $500\mu$S/cm.

When the difference between the electric conductivity D2 and the electric conductivity D1 is smaller than the predetermined dielectric conductivity Dm2 (step S13: N), the microcomputer 101M increments the count value N2 of the electric conductivity detection frequency counter by only "1" (step S14), and determines whether the count value N2 is equal to three times or more (step S15). When the count value N2 of the electric conductivity detection frequency is smaller than three times (step S15: N), the microcomputer 101M returns the processing flow to step S11 to detect the electric conductivity D2 again. Accordingly, even when the flow-in time of brine into the electrolytic bath 46 is delayed for some cause, the electric conductivity D2 can be surely detected. When the count value N2 of the electric conductivity detection frequency counter is equal to three times or more as a result of repetitive detection of the electric conductivity D2 (step S15: Y), the microcomputer 101M increments the count value N2 of the brine supply frequency counter by only "1" (step S16), and determines whether the count value N3 is equal to eight times or more (step S17).

When the count value N3 of the brine water supply frequency counter is smaller than eight times (step S17: N), the microcomputer 101M returns the processing flow to step S10 to supply brine water again. When the count value N3 of the brine water supply frequency counter is equal to eight times or more (step S17: Y), the microcomputer 101M determines that there is no brine in the brine tank 90, blinks the indicator lamp and stops the operation of the air filtering apparatus 1 (step S18).

When the difference between the electric conductivity D2 and the electric conductivity D1 is not less than the predetermined electric conductivity Dm2 (step S13: Y), it is determined that brine which is enough to execute the air filtering operation is supplied into the water receiving tray 42, the brine supply operation is finished, and the air filtering operation is resumed.

In the brine supply operation described above, brine is not supplied every time water is supplied to the water receiving tray 42, but brine is supplied only when the water exchange operation is carried out, that is, electrolysis of the water receiving tray 42 is discharged and then water is supplied to the water receiving tray 42. The execution of the brine supply operation of this embodiment is not limited to the above timing. However, according to the above operation, brine is not supplied when water is supplied to the water receiving tray 42 without discharging electrolytic water because the water level of the water receiving tray 42 is reduced or the like, and thus the control can be simplified.

Furthermore, the air filtering apparatus 1 is required to discharge water stocked in the water receiving tray 42 when the air filtering apparatus 1 is not used for a long term because of change of seasons or the like, when the installation place of the air filtering apparatus 1 is moved, when maintenance is carried out on the air filtering apparatus 1, etc. Therefore, a drain button (not shown) for accepting a drain operation for indicating discharge of water stocked in the water receiving tray 42 is provided to the operating panel 106.

When the drain button of the operating panel 106 is operated, the microcomputer 101M selects the maintenance operation mode. During the selection of the maintenance operation mode, the microcomputer 101M cleans the electrolytic water circulation passage, and then executes the maintenance operation of discharging water stocked in the water receiving tray 42. In connection with the start of the maintenance operation, the microcomputer 101M selects the water exchange operation mode described above irrespective of the accumulated operation time of the air filtering operation, and executes the brine supply operation. That is, the microcomputer 101M executes the water exchange operation, and then supplies brine to new water supplied to the water receiving tray 42. When the brine supply operation is finished, the microcomputer 101M circulates new water in which the chloride ion is increased, generates electrolytic water containing active oxygen species and then executes a cleaning operation of cleaning the electrolytic water circulation passage with the electrolytic water concerned. Subsequently, the microcomputer 101M discharges water stocked in the water receiving tray 42 to set the water receiving tray 42 to an empty state, and also in order to prevent breeding of various bacteria, etc. in the gas-liquid contact member 53, the microcomputer 101M drives the air blowing fan 31 for a fixed time so that air is blown to the gas-liquid contact member 53 to dry the gas-liquid contact member 53.

Through the above processing, in a case where the maintenance operation is started by user's drainage operation, the water exchange operation mode is selected even when the accumulated operation time of the air filtering operation does not reach a predetermined time, and brine is automatically supplied to the water receiving tray 42 after the water exchange operation. Accordingly, in the air filtering apparatus 1, even when tap water of a low chlorine concentration is supplied to the water receiving tray 42, brine is supplied to new water supplied to the water receiving tray 42, electrolytic water containing active oxygen species is generated, and the circulation passage is cleaned with this electrolytic water.

As described above, according to this embodiment, the pump 95 of the brine tank 90 is operated when the water exchange operation mode is selected, and brine is supplied to the water receiving tray 42. In this embodiment, brine is automatically supplied to new water supplied to the water receiving tray 42 after drainage. Therefore, the user does not feel troublesome, and the chlorine concentration which enables electrolysis can be secured.

Furthermore, according to this embodiment, brine is supplied to the water receiving tray 42 in accordance with the electric conductivity of water circulated in the water receiving tray 42 which is detected by the electric conductivity meter 49. Accordingly, brine is automatically supplied to the water receiving tray 42 in accordance with the electric conductivity of water circulated in the water receiving tray 42. Therefore, the user does not feel troublesome, and the chlorine concentration which enables electrolysis can be secured.

Still furthermore, according to this embodiment, in the water exchange operation, the drainage of water stocked in the water receiving tray 42 and the supply of tap water of a low chlorine concentration are repeated until the electric conductivity of water circulated in the water receiving tray 42 is equal to a predetermined value or less, and when the electric conductivity of water circulated in the water receiving tray 42 is equal to the predetermined value or less, the pump 95 of the brine tank 90 is operated to supply brine to the water receiving tray 42. Accordingly, after water stocked in the water receiving tray 42 is exchanged, brine is automatically supplied to the water receiving tray 42. Therefore, even when the water supplied to the water receiving tray 42 is tap water of a low chlorine concentration, electrolytic water containing hypochlorous acid whose concentration is enough to inactivate virus, etc. can be stably generated.

Furthermore, According to this embodiment, the air filtering operation mode is stopped every predetermined time, and after the water exchange operation mode is selected, the air filtering operation mode is resumed. Accordingly, electrolytic water which has been circulated and used is exchanged by new water every predetermined time, and condensation of electrolytic water and breeding of various bacteria, etc. can be prevented. Still furthermore, brine is automatically supplied to the water receiving tray 42 every time water stocked in the water receiving tray 42 is exchanged. Therefore, the user does not feel troublesome, and the chlorine concentration which enables electrolysis can be secured.

Second Embodiment (not according to the claimed invention)

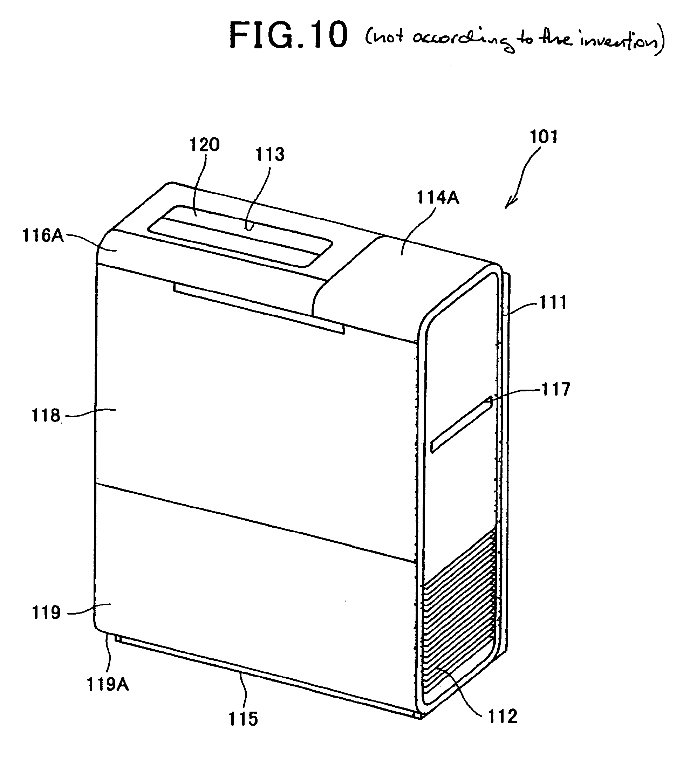

Fig. 10 is a perspective view showing the outlook of an air filtering apparatus 101 according to a second embodiment to which is basically the same as the outlook of the air filtering apparatus 1 according to the first embodiment of Fig. 1.

The air filtering apparatus 101 is an apparatus for sucking indoor air through a suction grille 112 and a suction port 115, filtering the indoor air and then discharging the filtered air from an air blow-out port 113, thereby cleaning the indoor air.

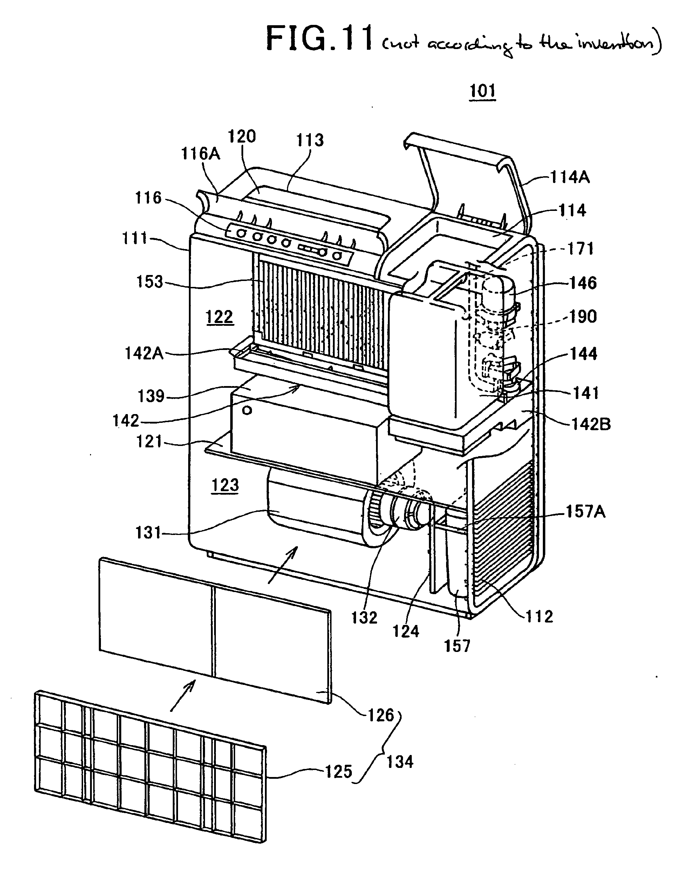

Fig. 11 is a perspective view showing the internal construction of the air filtering apparatus 101.

An operation lid 116A disposed at the front side of the air blow-out port 113 and a tank opening/closing lid 114A disposed abreast with the operation lid 116a are formed on the top surface of the housing 111. When the operation lid 116A is opened, an operating panel 116 for executing various operations of the air filtering apparatus 101 is exposed, and when the tank opening/closing lid 114A is opened, a water supply tank 141 described later can be taken in and out through a tank take-out port 114.

Gris portions 117 are formed at the upper portion of both the side surfaces of the housing 111. Each grid portion 117 is a recess portion to which a hand is hooked so that the housing 111 is carried by hands.

An upper cover member 118 and a lower cover member 119 are arranged in the vertical direction on the front surface (one side surface) of the housing 11 so as to be freely detachable, and the internal construction of the housing 111 is exposed by detaching the upper cover member 118 and the lower cover member 119. The lower cover member 119 has an arc portion 119A curved toward the back side of the housing 111 at the lower end portion of the lower cover member 119, and the suction port 115 is formed at the arc portion 119A.

As shown in Fig. 11, a support plate 121 for partitioning the inside of the housing 111 into upper and lower chambers 122 and 123 is provided in the housing 111. An air blowing fan 131 and a fan motor 132 are disposed in the lower chamber 123, and a drain tank 157 having a grip portion 157A is accommodated through the partition plate 124 so as to be pulled out to the front side of the housing 111. The air blowing fan 131, the fan motor 132 and the drain tank 157 are disposed abreast with one another.

Furthermore, a pre-filter 134 is freely detachably disposed between the air blowing fan 131 and the suction port 115, that is, such a position as to face a lower cover member 119 (Fig. 10) in the lower chamber 123. The pre-filter 134 comprises a rough dust filter 125 for collecting large particle-size dusts, etc. in the air sucked through the suction grilles 112 and the suction port 115, and a second filter 126 for collecting dusts, etc. which are passed through the rough dust collector 125 and have particle diameters of about 10($\mu$m), for example. Grass pollen, dusts, etc. floating in the air are removed by the pre-filter 134, and the air from which the grass pollen, dusts, etc. are removed is supplied through the air blowing fan 131 into the upper chamber 122.

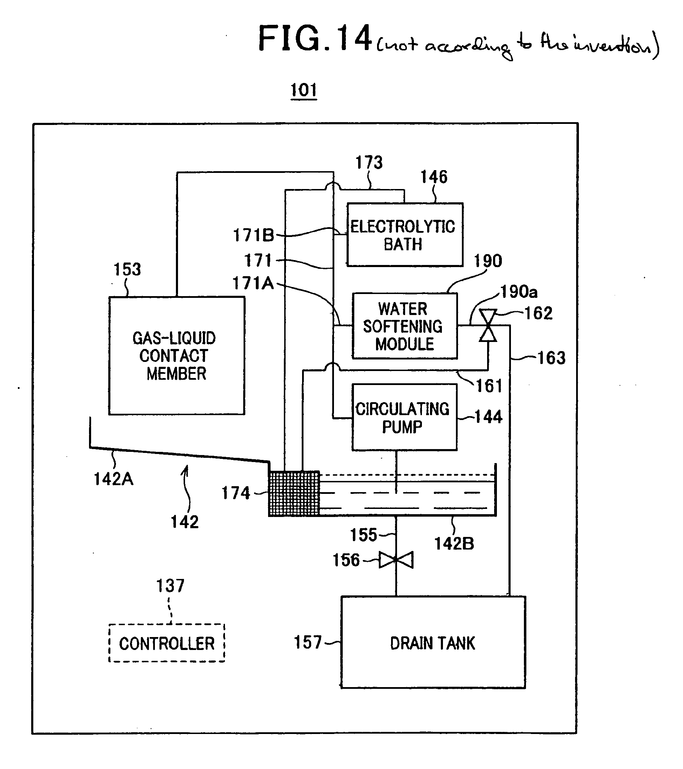

An electrical component box 139 is disposed at the upper side of the air blowing fan 131 and the fan motor 132 is disposed in the upper chamber 122, and a control board on which various kinds of devices constituting a controller 137 (Fig. 14) for controlling the air filtering apparatus 1 are mounted, various kinds of electrical component parts such as a power supply circuit for supplying a power supply voltage to the fan motor 132, etc. are accommodated in the electrical component box 139. The controller 137 controls a louver 120, a fan motor 132, etc. in accordance with the operation of the operating panel 116.

A gas-liquid contact member 153 for bringing passing air into contact with electrolytic water to filter the air is disposed at the upper side of the electrical component box 139. A water receiving tray 142 having a water receiving unit 142A for receiving electrolytic water dropping from the gas-liquid contact member 153 is disposed at the lower side of the gas-liquid contact member 153. The water receiving tray 142 has a stock unit 142B formed at the deep bottom, and the stock unit 142B can stock much electrolytic water. The stock unit 142B is designed so that electrolytic water dropping from the water receiving unit 142A flows into the stock unit 142B, and electrolytic water is stocked in the stock unit 142B. Furthermore, the stock unit 142 extends to the upper side of the drain tank 157.

Furthermore, a water supply tank 141 is disposed at the upper side of the stock unit 142B, and water can be supplied from the water supply tank 141 to the stock unit 142B>

Furthermore, a circulating pump 144 for supplying electrolytic water to the gas-liquid contact member 153, a water softening module 190 for reducing the hardness of electrolytic water and an electrolytic bath 146 for generating electrolytic water are provided at the upper side of the stock unit 142B.

The air flow in the air filtering apparatus 1 will be described.

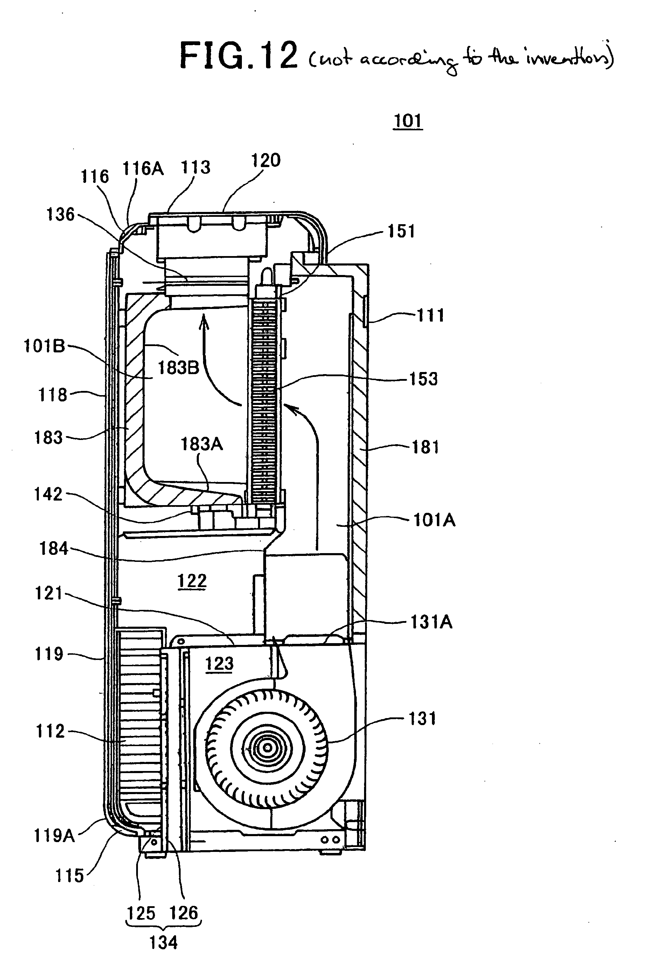

Fig. 12 is a side cross-sectional view of the internal construction of the air filtering apparatus 1.

As described above, an air blowing fan 131 is provided in the lower chamber 123 of the housing 111. The air flow port 131A of the air blowing fan 131 is provided face-up at the back-side portion of the housing 111, and intercommunicates with a space 101A as a flow passage extending vertically at the back side of the upper chamber 122. The space 101A is defined by a first air guide member 181 disposed at the back side of the housing 111, and an air guide plate 184 which is disposed to face the first air guide member 181 and extends from the support plate 121 to the water receiving tray 142. Air blown out from the air flow port 131A of the air blowing fan 131 passes through the space 101A as indicated by an arrow of Fig. 12, and sprayed to the back side of the gas-liquid contact member 153.

A second air guide member 183 for guiding air passing through the gas-liquid contact member 153 to an air blow-out port 113 is disposed in a space 101B at the opposite side to the space 101A with respect to the gas-liquid contact member 153 (at the front side of the housing 111 in the second embodiment) as shown in Fig. 12. The second air guide member 183 is formed to have a substantially box-like shape opened at the upper portion and the back portion thereof.

The second air guide member 183 has not only a function of guiding air in the space 101B to the air blow-out port 113, but also a function of receiving air passing through the gas-liquid contact member 153 and water sprayed to this space 101B (so-called spattering water). Specifically, the inner bottom surface 133A of the second air guide member 183 is inclined to the gas-liquid contact member 153, and water spattering to the second air guide member 183 is guided to the water receiving tray 142. Air passing through the gas-liquid contact member 153 is guided to the inner surface 183B of the second air guide member 183, passes through an air blow-out port filter 136 disposed at the lower side of the air blow-out port 113, and then exhausted.

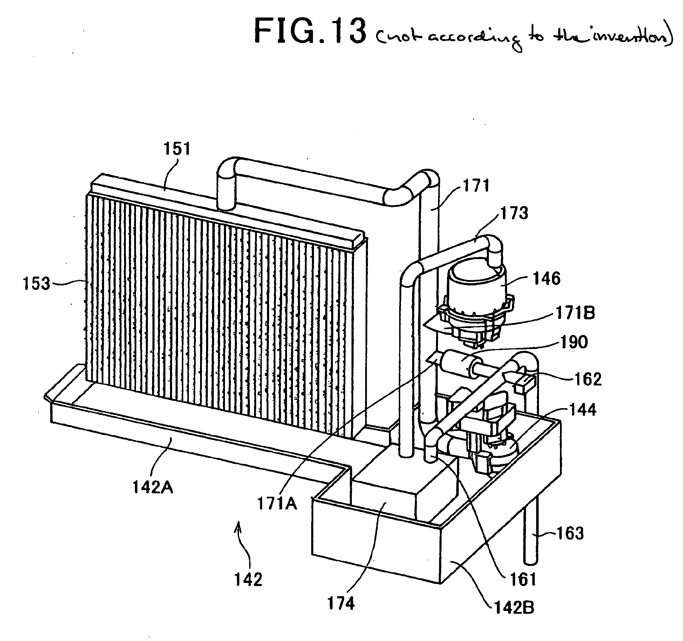

Fig. 13 is a perspective view showing the construction of a main part of generating/circulating electrolytic water in the air filtering device 101.

In the air filtering apparatus 101, electrolytic water for filtering air is circulated and repetitively used, whereby air is filtered over a long term by effectively using a small amount of water. That is, in the air filtering apparatus 1, electrolytic water is stocked in the stock unit 142B, and pumped up from the stock unit 142B by the circulating pump 144. A part of the electrolytic water pumped up from the stock unit 142B by the circulating pump 144 is supplied to the gas-liquid contact member 153, infiltrated into the gas-liquid contact member 153 and brought into contact with air to filter the air. Thereafter, the electrolytic water is returned to the stock unit 142B, and supplied to the gas-liquid contact member 153 by the circulating pump 144 again to be repetitively used for air filtering.

The gas-liquid contact member 153 is disposed above the water receiving unit 142A and used for air filtering, and electrolytic water dropping from the gas-liquid contact member 153 is received by the water receiving unit 142A.

The water receiving tray 142 is constructed by forming the water receiving unit 142A and the stock unit 142B integrally with each other. The water receiving unit 142A is formed so as to be located at a one-step higher position than the stock unit 142B. The electrolytic water dropping from the gas-liquid contact member 153 to the water receiving unit 142A flows to the stock unit 142B. An electrolytic water flow path extending from the water receiving unit 142A to the stock unit 142B is provided with a filter 174 for collecting solid materials (scale) contained in water dropping from the gas-liquid contact member 153.

The circulating pump 144 is disposed so that the suction port thereof is lower than the water level of the stock unit 142B, and discharges electrolytic water through a water distribution pipe 171 connected to the discharge port of the circulating pump 144. The water distribution pipe 171 is a pipe for connecting the circulating pump 144 and the gas-liquid contact member 153. A first branch pipe 171A and a second branch pipe 171B are branched from the water distribution pipe 171. The first branch pipe 171A is connected to the water softening module 190, and the second branch pipe 171B which is branched at the further downstream side of the first branch pipe 171A is connected to the electrolytic bath 146. Electrolytic water is supplied from the circulating pump 144 to the water softening module 190 and the electrolytic bath 146.

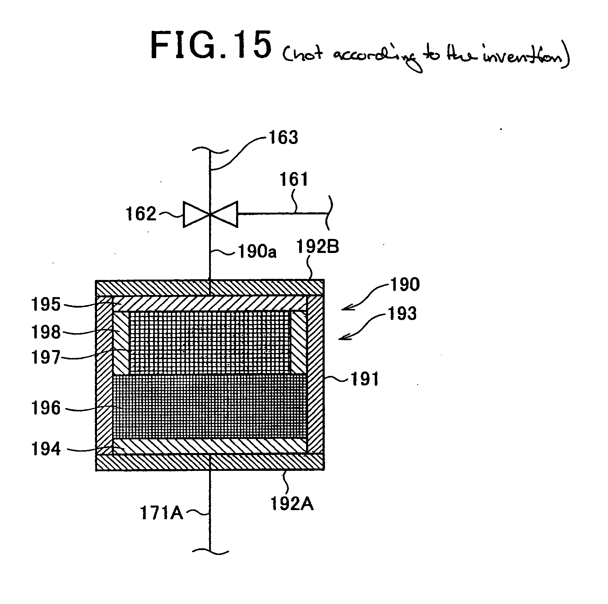

The water softening module 190 executes a treatment of softening electrolytic water passed through the first branch pipe 171A. The water softening treatment is a treatment of reducing the concentration of hard components such as calcium ion ($Ca^{2+}$), magnesium ion ($Mg^{2+}$), etc. contained in water to reduce the hardness of water. The water softening module 190 contains a pair of electrodes 194 and 195 (Fig. 15) described later, and applies a voltage between the electrodes 194 and 195 to reduce the hardness of electrolytic water.

A three-way valve 162 is connected to the electrolytic water discharge port 190a of the water softening module 190. A softened water return pipe 161 (first discharge pipe) for returning softened electrolytic water to the stock unit 142B is connected to one exit of the three-way valve 162, and a drain pipe 163 (second discharge pipe) connected to the drain tank 157 is connected to the other exit of the three-way valve. The three-way valve 162 is switched under the control of the controller 137.

In the electrolytic bath 146, electrolytic water passing through the second branch pipe 171B is electrolyzed. As in the case of the first embodiment, the electrolytic bath 146 contains the pair of electrodes 47 and 48 (Fig. 7B), and a voltage controlled by the controller 137 is applied between the electrodes 47 and 48 to electrolyze water and thus generate electrolytic water. In the second embodiment, by electrolyzing tap water, electrolytic water containing hypochlorous acid (HClO) as an air filtering component is generated. An electrolytic water discharging pipe 173 for feeding out electrolytic water to the stock unit 42B is connected to the discharge port of the electrolytic bath 46.

The terminals of the water softening return pipe 161 and the electrolytic water discharge pipe 173 are located above the filter 174, and electrolytic water flowing out from the water softening module 190 and the electrolytic bath 146 is directly poured from the terminals of the water softening return pipe 161 and the electrolytic water discharge pipe 173 into the filter 174. When the electrolytic water concerned is passed through the filter 174, scale, etc. are removed from the electrolytic water by the filter 174, and then the electrolytic water concerned is returned to the stock unit 142B.

Therefore, even when scale occurs in the water softening module 190 and the electrolytic bath 146 and the scale concerned flows together with electrolytic water, the scale is collected by the filter 174, and thus scale can be prevented from flowing into the electrolytic water circulation passage containing the gas-liquid contact member 153.

As in the case of the first embodiment, electrolytic water containing hypochlorous acid generated in the electrolytic bath 146 is stocked in the stock unit 142B, and supplied from the stock unit 142B to the gas-liquid contact member 153 by the circulating pump 144. When air is blown to the gas-liquid contact member 153 by the air blowing fan, virus, etc. floating in the air are brought into contact with the electrolytic water to inactivate virus, etc. when the air passes through the gas-liquid contact member 153, whereby the air can be filtered. In addition to the air filtering effect, electrolytic water has an effect of preventing breeding of various bacteria, etc. in the gas-liquid contact member 153 itself. Furthermore electrolytic water containing hypochlorous acid ionizes odor when the odor passes through the gas-liquid contact member 153, whereby the odor is dissolved in the electrolytic water and thus can be removed from the air. That is, the electrolytic water has a deodorizing effect.

Here, as in the case of the first embodiment, a water sprinkling box 151 for uniformly dispersing electrolytic water onto the gas-liquid contact member 153 is installed to the upper portion of the gas-liquid contact member 153. The water sprinkling box 151 has a tray member (not shown) for temporarily stocking electrolytic water, and plural water sprinkling holes (not shown) are formed in the side surface of the tray member. Electrolytic waster supplied through the water distribution pipe 171 is dropped from the water sprinkling holes to the gas-liquid contact member 153.

Furthermore, the gas-liquid contact member 153 has a filter having a honeycomb structure. In detail, the gas-liquid contact member 153 has a structure that an element portion coming into contact with air is supported by a frame. The element portion is constructed by laminating a corrugated plated member and a flat plate member, and a number of substantially triangular openings are formed between the corrugated plate member and the flat plate member. Accordingly, a large gas contact area can be secured when air is passed through the element portion, electrolytic water can be dropped through the element portion, and thus the element portion hardly clogs.

Furthermore, in order to efficiently disperse electrolytic water dropping from the water sprinkling box 151 to the element portion, a water distribution sheet (not shown) is disposed on the top surface of the gas-liquid contact member 153. This water distribution sheet is a sheet (woven fabric, non-woven cloth or the like) formed of fibrous material having liquid permeability, and one or plural sheets are provided along the section in the thickness direction of the gas-liquid contact member 153.

Furthermore, the respective parts (containing the frame, the element portion and the water distribution sheet) of the gas-liquid contact member 53 are formed of materials which are not deteriorated by electrolytic water, for example, polyolefin resin (polyethylene resin, polypropylene resin or the like), PET (polyethylene terephthalate) resin, vinyl chloride resin, fluorinated resin (PTFE, PFA, ETFE or the like), ceramic material of the like. In this embodiment, PET resin is used.

Furthermore, the respective parts of the gas-liquid contact member 153 are subjected to a hydrophilic treatment to enhance the affinity to electrolytic water. Accordingly, water retentivity of the gas-liquid contact member 53 to electrolytic water (wettability) is kept, and the contact between active oxygen species (active oxygen materials) described later and indoor air is kept for a long term.

Here, the supply of electrolytic water to the gas-liquid contact member 153 is performed in the same manner as the first embodiment. In the second embodiment, a case where tap water is poured in the water supply tank 141 (Fig. 11) and the air filtering apparatus 101 is operated will be described.

When the water supply tank 141 filled with tap water is set in the air filtering apparatus 101, tap water is supplied from the water supply tank 141 into the stock unit 142B as described above, and the water level of the stock unit 14B reaches a predetermined level. Water in the stock unit 142 is pumped up by the circulating pump 144, and a part of the electrolytic bath 146 is supplied. As shown in Fig. 7A, the electrolytic bath 146 is provided with a pair of electrodes 47 and 48, one electrode serving as an anode and the other electrode serving as a cathode. By applying a voltage between these electrodes 47 and 48, tap water flowing into the electrolytic bath 146 is electrolyzed, and electrolytic water containing active oxygen species is generated.

Here, the active oxygen species means oxygen having higher oxidation activity than normal oxygen and relevant materials thereto. For example, it contains so-called narrowly-defined active oxygen such as superoxide anion, singlet oxygen, hydroxyl radical, hydrogen peroxide, etc. and so-called broadly-defined active oxygen such as ozone, hypohalous acid, etc. as described above with respect to the first embodiment. Furthermore, each of the electrodes 47 and 48 comprises a base of titan (Ti) and a coating layer of iridium (Ir), platinum (Pt), and the current value flowing through the electrodes 47 and 48 is set to be equal to several mA (milliamperes)/cm$^2$ (square centimeter) to several tens mA/cm$^2$ in current density, and a predetermined free residual chlorine concentration (for example, 1mg (milligrams)/1 (liter)) is generated. The detailed reaction of the electrodes 47 and 47 is the same as the first embodiment, and thus the description thereof is omitted.

Fig. 14 is a schematic diagram showing the electrolytic water circulation passage.

The water supply tank 141 (Fig. 11) is disposed in the stock unit 142B so that a water supply port thereof faces the bottom of the stock unit 142B, and a float valve is provided to the water supply port so that the float valve is opened when the water level of the stock unit 142B is lower than the water supply port. Accordingly, a required amount of water is supplied from the water supply tank 141 is supplied, and the water level of the stock unit 142B is kept constant.

Furthermore, a drainpipe 155 for connecting the stock unit 142B to a drain tank 157 for stocking discharged electrolytic water is provided to the bottom portion of the stock unit 142B, and a drain valve 156 for controlling drainage of the stock unit 142B is secured to the drain pipe 155. When the drain valve 156 is opened, electrolytic water is discharged from the stock unit 142B to the drain tank 157, the float valve of the water supply tank 141 is actuated and new water is supplied into the stock unit 142B.

In the air filtering apparatus 101, electrolytic water stocked in the stock unit 142B is pumped up by the circulating pump 144 and branched in three directions so as to reach the gas-liquid contact member 153, the water softening module 190 and the electrolytic bath 146, and then returned to the stock unit 142B, thereby repeating the circulation.

That is, the electrolytic water in the stock unit 142B is circulated in the gas-liquid contact member 153 while subjected to the water softening and electrolysis treatments stationarily, and used for air filtering. Electrolytic water which is softened by the water softening module 190 to be reduced in hardness flows through the circulation passage going out from the stock unit 142B and returning to the stock unit 142B again.

The electrolytic water softened by the water softening module 190 can be directly drained to the drain tank 157 by switching the three-way valve 162 under the control of the controller 137.

The scale described above occurs due to the following causes. Water such as tap water or the like which is supplied to the electrolytic bath 146 as a raw material of electrolytic water contains hardness components (calcium ion, magnesium ion, etc.) in most cases. When the air filtering operation is executed for a long term, particularly in the gas-liquid contact member 153 in which electrolytic water is easily vaporized, these hardness components are condensed due to vaporization of electrolytic water, and thus precipitate as scale. When scale adheres to the surface of the gas-liquid contact member 153, the gas-liquid contact member 153 is clogged, the water retentivity of electrolytic water in the gas-liquid contact member 153 is lowered or the like, so that the contact between air and electrolytic water is disturbed and thus the air filtering efficiency may be lowered. Furthermore, when scale adheres to the electrodes 47, 48 of the electrolytic bath 146 or when the pipes are clogged with scale, the air filtering efficiency may be lowered because the supply amount of electrolytic water is deficient or the like.

In the second embodiment, occurrence of scale is suppressed by using the water softening module 190 for softening electrolytic water by reducing the hardness of the electrolytic water.

Fig. 15 is a schematic diagram showing the construction of the water softening module 190.

The water softening module 190 has a case body 193 constructed by fixing lid members 192A and 192B to both the ends of a cylindrical case 191. The water softening module 190 is constructed by accommodating a pair of electrodes 194 and 195, a fabric body 196 (collecting member) having a collecting function, an insulating spacer 197 and a fixing ring 198 for holding the spacer 197 in the case body 193.

A first branch pipe 71A is connected to the lid member 192A, and a three-way valve 162 is connected to the discharge port of electrolytic water in the lid member 192B. That is, electrolytic water flows in from one lid member 192A side, and flows out from the other lid member 192B side. The case body 193 is formed of an insulating resin material. The lid members 192A and 192B are detachable from the case 191.

Electrodes 194 and 195 are disc-shaped electrodes, and secured so that the peripheries thereof abut against the inner peripheral surface of the case 191. The electrodes 194 and 195 are located at both the ends of the inside of the case body 193, the electrode 194 is disposed at the flow-in side of electrolytic water and the electrode 195 is disposed at the flow-out (discharge) side of electrolytic water. The electrodes 194 and 195 are designed like a mesh, and thus has a water-permeable property. Accordingly, electrolytic water passes through the electrodes 194 and 195.

Furthermore, the controller 137 controls the voltage applied between the electrodes 194 and 195 on the basis of power supplied from a DC power source (not shown) so that the value of current flowing between the electrodes 194 and 195 is equal to a fixed value (constant current).

Here, the electrodes 194 and 105 are platinum-iridium coated titan electrodes obtained by coating titan electrodes with alloy formed of platinum and iridium. For example, single substance such as platinum, iridium, tantalum, palladium, titan, stainless or the like, or an insoluble electrical conductive material containing at least some of these metal materials may be used as the material of the electrodes 194 and 195.

The fabric body 196 is an assembly of carbon fibers having electric conductivity, and formed in a disc-shape. The fabric body 196 is secured to a surface of the electrode 194 at the downstream side of electrolytic water, and the outer peripheral surface thereof is in contact with the inner peripheral surface of the case 191. The fabric body 196 is an assemble of fibers and has a large surface area, so that a large amount of foreign materials such as scale, etc. can be caught and collected on the surface of the fabric body 196. In addition, the fabric body 196 has many voids and thus it has the water-permeable property.

Here, carbon fiber is used as the material of the fabric body 196. It may be formed of activated carbon fiber, platinum fiber, titan fiber or carbon nanotube, or any one or at least two kinds or more of carbon fiber, resin fiber (resin fiber having electric conductivity by itself such as polyacetylene resin doped with iodine, arsenic pentafluoride or the like or the like, resin fiber blended with electrical conductive material as a composition), activated carbon fiber and titan fiber which is coated with catalysis.

The spacer 197 is an insulating resin member designed in a disc-shape. The spacer 19 has a porous structure and the water-permeable property, and can catch and collect foreign materials such as scale, etc. therein. As an example, the void ratio of the spacer 197 is equal to 95%. The void ratio means a rate at which the volume of the void portions existing in the space occupy. The spacer 197 may be designed in a mesh-shape.

The spacer 197 is pinched by the fabric body 196 and the electrode 195 at the downstream side of the flow of the electrolytic water in the fabric body 196. The fabric body 196 is pressed by the spacer 197, and slightly deformed. That is, the spacer 197 prevents current flow caused by the contact between the fabric body 196 and the electrode 195, and presses the fabric body 196 against the electrode 194 side to reduce the electrical resistance (contact resistance) between the electrode 194 and the fabric body 196.

The fixing ring 198 is a ring-shaped insulating member. The fixing ring 198 is held in the case 191 so that no gap occurs between the outer peripheral surface of the fixing ring 198 and the inner peripheral surface of the case 191. the spacer 197 is held inside the fixing ring 198.

Next, the water softening treatment of the water softening module 190 will be described.

The water softening module 190 of the air filtering apparatus 101 switches a water softening operation mode of reducing the hardness of electrolytic water and a cleaning operation mode in which a voltage is applied to the electrodes 194 and 195 while the polarities of the electrodes 194 and 195 when the water softening operation mode is executed are reversed, and executes the selected operation mode. The details will be described later, however, the cleaning operation mode is an operation of removing scale components adhering to the water softening module 90.

The water softening operation mode is a mode for softening electrolytic water, and it is executed during air filtering operation of the air filtering apparatus 101.

When the start of the air filtering operation is instructed to the air filtering apparatus 101, the controller 137 starts the fan motor 132, etc., and also starts the circulating pump 144, the water softening module 190 and the electrolytic bath 146, whereby the air filtering operation of circulating electrolytic water in the circulation passage containing the gas-liquid contact member 153 is started.

In the water softening module 190, the controller 137 applies a voltage to the electrodes 194 and 195, the electrode 194 is set to a cathode (negative potential) and the electrode 195 is set to an anode (positive potential). The fabric body 196 which is brought into contact with the electrode 194 and electrically connected to the electrode 194 is set to a cathode (negative potential).

Accordingly, at the fabric body 196 which is located at the upstream side of the flow of the electrolytic water in the water softening module 190 and serves as the cathode, hydrogen ion ($H^+$) and hydroxide ion ($OH^-$) in water react with each other according to the following formula:

$$4H^+ + 4e^- + (4OH^-) \rightarrow 2H_2 + (4OH^-)$$

At the electrode 195 which is located at the downstream side and serves as the anode, water is electrolyzed according to the following formula:

$$2H_2O \rightarrow 4H^+ + O_2 + 4e^-$$

As described above, at the fabric body 196 serving as the cathode as described above, hydroxide ion ($OH^-$) is generated. Hydroxide ion is very strong base and thus the negatively-charged surface of the fabric body 196 is locally alkaline. Accordingly, the hardness components in electrolytic water react with hydroxide ion, and it becomes a base. Specifically, ions of calcium, magnesium, potassium, silica, etc. which are main scale components contained in electrolytic water become hardly soluble salts such as calcium hydrate, calcium carbonate and magnesium hydrate, etc. and are precipitated. Furthermore, when ions of phosphorus, sulfur, zinc, etc. are contained, salts such as calcium sulfate, calcium sulfite, calcium phosphate, zinc phosphate, zinc hydrate, basic zinc, etc. may be precipitated. Ions of calcium, magnesium, potassium, silica, etc. which are scale components are precipitated as crystalline bodies on the fabric body 196 by electrodeposition action. Particularly, the fabric body 196 has a large surface area, and deposition of a large amount of crystal bodies occurs on the fabric body 196, and thus the fabric 196 has a high capability of collecting crystal bodies. Deposition materials obtain by deposition of hardness components such as calcium ion, magnesium ion, etc. occurring as scales on the fabric bodies 196 through execution of the water softening operation mode will be referred to as "crystal bodies".

As described above, in the water softening operation mode, when electrolytic water passes through the water softening module 190, deposition of the hardness components such as calcium ion, magnesium ion, etc. in the electrolytic water occurs in the fabric body 196 in the electrolytic circulation passage along which the electrolytic water goes out from the stock unit 142B and returns to the stock unit 142B again. Therefore, the concentration of the hardness components contained in the circulated electrolytic water is lowered, and thus the electrolytic water is softened.

Furthermore, in the water softening operation mode, the crystal bodies precipitated on the fabric body 196 are promoted to be peeled off by water stream flowing from the electrode 194 side to the electrode 195 side. Therefore, the crystal bodies can be prevented from being excessively grown in the fabric body 196, and the crystal bodies flowing to the downstream side can be withdrawn by the spacer 197. The spacer 197 has the porous structure as described above, and thus a large amount of crystal bodies can be withdrawn.

A general index for indicating the hardness of water is calculated according to the following formula (1), for example.

$$\textsf{Hardness = calcium amount x 2.5 + magnesium amount x 4.1}$$
$$\textsf{... (1)}$$

Here, the unit of the hardness, the calcium amount and the magnesium amount is (mg/l), and the hardness calculated by the above formula (1) is a value obtained by converting the amount (mg) of calcium and magnesium contained in water of 1 liter to the amount (mg) of calcium carbonate. When the hardness calculated from the formula (1) exceeds 300 to 400(mg/l), deposition of calcium and magnesium easily occurs (calcium and magnesium are easily precipitated).

However, by using the water softening module 90 of the second embodiment, the hardness of electrolytic water can be lowered to 200(mg/l) or less, for example, so that deposition of scale can be suppressed.

Next, the cleaning operation mode will be described.

By processing electrolytic water in the water softening operation mode, the crystal bodies are precipitated in the fabric body 196, and when a large amount of crystal bodies are precipitated with lapse of the operation time, it may affect the efficiency of the water softening of the water softening module 190. Therefore, it is desired to remove the crystal bodes precipitated in the fabric body 196.

In the cleaning operation mode, the crystal bodies precipitated in the fabric bodies 196 in the water softening module 190 is dissolved and exfoliated, and the dissolved and exfoliated crystal bodies are discharged to the drain tank 157. In the second embodiment, the cleaning operation is executed every time the accumulated operation time of the water softening operation mode reaches a predetermined time.

In the cleaning operation mode, a voltage is applied to the electrodes 194 and 195 while the polarities of the electrodes 194 and 195 in the water softening operation mode are reversed. That is, the fabric body 196 is set to the anode (positive potential), and the electrode 195 at the downstream side is set to the cathode (negative potential).

In this case, the surface of the fabric body 196 is locally inclined to acidity through the electrolysis reaction, and the crystal bodies precipitated on the surface concerned and a part or the whole of the crystal bodies adhering to the spacer 197 are dissolved into anode ions. The crystal body is easily peeled off from the adhering surface of the fabric body 196 when it is partially dissolved. Therefore, the crystal bodies are discharged to the drain tank 157 together with the electrolytic water flowing in the drain pipe 63.

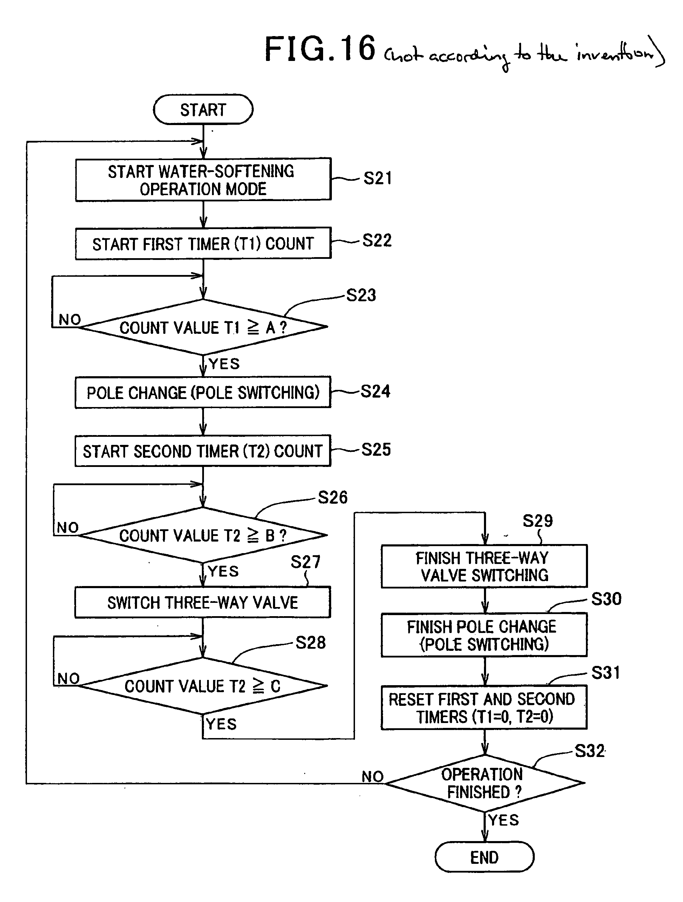

Fig. 16 is a flowchart showing the operation of the cleaning operation mode.

The operation shown in Fig. 16 is started together with start of the air filtering operation, for example. When the operation of Fig. 16 is executed, the controller 137 executes a counting operation based on two timers which are implemented by hardware or software. These two timers (first timer, second timer) are timers which count up at a predetermined period with time lapse, and each of the timers can start/stop the time count independently.

When starting the water softening operation mode together with start of the air filtering operation (step S1), the controller 137 starts the count of the first timer for counting an accumulated actuation time of the water softening operation mode (step S2). Thereafter, the controller 37 refers to the count value T1 of the first timer, and executes the water softening operation mode until the timer count value T1 reaches a preset value A (step S3).

Here, the set value A sets the accumulated actuation time of the water softening operation mode and the start timing of the cleaning operation mode, and determines an execution frequency of the cleaning operation mode. As described above, crystal bodies accumulate in the water softening module 190 together with the time by the execution of the water softening operation mode as described above. However, the water softening function of the water softening module 190 may be gradually lowered together with increase of the crystal bodies. Therefore, it is preferable to determine the set value A so that the cleaning operation mode is executed frequently to the extent that the water softening function of the water softening module 190 is not lowered to a practical level or less. As a specific example, the set value A is set to the count value corresponding to five hours of the actual time.

When the count value T1 of the first timer reaches the set value A (step S3: Yes), the controller 137 switches the polarities of the electrodes 194, 195 to the opposite polarities to those in the water softening operation mode and then applies the voltage (change the polarities) (step S4), starts the count of ht second timer (step S5) and starts the cleaning operation mode. In this cleaning operation mode, by reversing the polarities of the electrodes 194 and 195, the crystal bodies precipitated in the water softening module 190 are dissolved and exfoliated. Furthermore, the cleaning operation mode is executed in parallel to the air filtering operation, and the circulating pump 144 is also actuated during even the cleaning operation mode. Therefore, the dissolved and exfoliated crystal bodies flow into the stock unit 142B together with electrolytic water, and collected by the filter 174.

The controller 137 continues the cleaning operation mode until the count value T2 of the second timer reaches a preset set value B (step S6). When the count value T2 reaches the set value B (step S6: Yes), the three-way valve 62 is switched so that electrolytic water flows from the water softening module 190 to the drain pipe 163 (step S7), and discharge of the electrolytic water to the drain tank 157 is started. Through the operation of this step S7, the crystal bodies dissolved and exfoliated in the water softening module 190 are discharged to the drain tank 157 together with the electrolytic water.

Here, the set value B is a set value for determining a time period from the time when the cleaning operation mode is started until the time when the three-way valve 162 is switched to start the drainage, and it is preferable to set the set value B to such a time that a part of the whole of the crystal bodies precipitated in the water softening module 190 can be dissolved. As a specific example, the set value B is set to the count value corresponding to nine minutes and forty seconds of the actual time. The second timer continues the count even after the count value T2 reaches the set value B.

Subsequently, the controller 137 is on standby until the count value T2 of the second timer reaches a preset set value C under the state that the electrolytic water in the water softening module 90 is discharged to the drain tank 157 (step S10). Here, when the count value T2 reaches the set value C (step S8: Yes), the controller 137 returns the switch state of the three-way valve 162 to the original state so that electrolytic water flows to the softened water return pipe 161 again (step S9), finishes the change of the polarities of the electrodes 194 and 195 and returns the polarities to the same polarities in the water softening operation mode (step S10).

The set value C is a value for determining a time period from the step S7 to the time when the three-way valve 162 is switched to discharge the electrolytic water to the drain tank 157, and it is preferable that the set value C is set to such a time that most of the crystal bodies in the water softening module 190 can be discharged. The discharge time of the electrolytic water corresponds to the time period from the time when the count value T2 of the second timer reaches the set value B till the time when the count value T2 reaches the set value C. Therefore, the set value C corresponds to the sum of the discharge time and the set value B. For example, when the set value B is set to the value corresponding to nine minutes and forty seconds of the actual time, in order to discharge the electrolytic water from the water softening module 190 for 20 seconds, the set value C is set to the count value corresponding to 10 minutes (actual time) obtained by adding 9 minutes 40 seconds with 20 seconds.

After the switch state of the three-way valve 162 and the polarity-changed state of the electrodes 194, 195 are returned to the original states, the controller 137 resets the count value T1 of the first timer and the counter value T2 of the second timer (step S11), and determines whether the operation should be finished or not (step S12). When the operation is continued, the processing returns to step S1. When it is instructed to finish the air filtering operation or the water softening operation mode (step S12: Yes), the operation of Fig. 16 is finished.

When it is instructed to finish the air filtering operation or the water softening operation mode by the operation of the operating panel 116 or the like during execution of the operation of Fig. 16, the controller 137 may immediately stop each unit and finish the operation of Fig. 16, or may continue the operation till the step S12 and then stop each unit.

As described above, the crystal bodies precipitated on the surface of the fabric body 196 in the water softening operation mode are dissolved and exfoliated to be removed by the cleaning operation mode in which the polarities of the electrodes 194 and 195 are reversed. Furthermore, the exfoliation of the crystal bodies is physically further promoted by the water stream flowing in the water softening module 190 during the cleaning operation mode. Accordingly, the crystal bodies precipitated in the water softening operation mode can be easily removed from the fabric body 196, so that the water softening function of the water softening module 190 can be kept to an excellent state at all times and the hardness of electrolytic water can be lowered.

Furthermore, by properly setting the set values B and C, the discharge amount of the electrolytic water can be easily adjusted, and the discharge amount of electrolytic water can be suppressed to a required sufficient amount, so that water can be saved. Furthermore, the frequency of the water supply work to the water supply tank 141 can be lowered and the continuous operation can be performed for a long term. Still furthermore, the three-way valve 162 is switched to the drain pipe 163 side under the control of the controller 137, and electrolytic water containing crystal bodies is discharged to the drain tank 57, so that a large amount of crystal bodies can be prevented from being discharged to the electrolytic water circulation passage, and adherence of the crystal bodies to the circulation passage, etc. can be prevented. In addition, the crystal bodies flowing to the stock unit 142B side during execution of the cleaning operation mode are collected by the filter 174, so that the adherence of the crystal bodes to the electrolytic water circulation passage, etc. can be surely prevented.

Furthermore, in the water softening module 190, the fabric body 196 is provided at the upstream side of the flow of the electrolytic water, and the spacer 197 is provided at the downstream side of the electrolytic water flow, so that the exfoliated crystal bodies can be efficiently collected by the spacer 197 at the downstream side. Accordingly, a large amount of crystal bodies can be efficiently collected.

When a large amount of crystal bodies are accumulated by the spacer 197 and thus it is required to clean or exchange the spacer 197, the spacer 197 can be easily taken out by detaching the lid members 192A and 192B.

As described above, according to the second embodiment electrolytic water which is circulated in the electrolytic water circulation passage containing the gas-liquid contact member 153 is lowered in hardness through the water softening treatment of the water softening module 190. Accordingly, the hardness components such as calcium, magnesium, etc. contained in electrolytic water are hardly precipitated as scales in the circulation passage containing the gas-liquid contact member 153 (i.e., deposition of the hardness components in the circulation passage hardly occurs). Particularly, in the construction that electrolytic water is circulatingly supplied to the gas-liquid contact member 153, the hardness components are condensed due to vaporization of the electrolytic water or the like, and deposition of scales trends to occur easily. However, by lowering the hardness of the electrolytic water in the water softening module 190, the deposition of scales can be suppressed. Degradation of the water retentivity of the gas-liquid contact member 153 and clogging of the pipes can be prevented by suppressing the deposition of scales, so that the air filtering efficiency can be kept excellent.

Electrolytic water stocked in the stock unit 142B is fed to the water softening module 190 through the operation of the circulating pump 144 and softened in the water softening module 190, and the hardness-lowered electrolytic water is returned from the water softening module 190 to the stock unit 142B. Accordingly, the hardness-lowered electrolytic water is surely supplied to the electrolytic water circulation passage containing the gas-liquid contact member 153, and the deposition of scales can be suppressed in the overall circulation passage.

Furthermore, electrolytic water is pumped up from the stock unit 142B and softened in the water softening module 190, and then returned to the stock unit 142B again. Therefore, the water softening module 190 can be easily installed.

Electrolytic water stocked in the stock unit 142B is supplied to the water softening module 190 by the function of the circulating pump 144, and thus it is unnecessary to provide a new pump for supplying the electrolytic water to the water softening module 190. Therefore, electrolytic water can be supplied to the water softening module 190 with a simple construction.

Furthermore, the three-way valve 162 is provided, and switched under the control of the controller 137, whereby electrolytic water discharged from the water softening module 190 can be discharged to any one of the stock unit 142B and the drain tank 157 while the three-way valve 126 is switched to one of the stock unit 142B and the drain tank 157. Therefore, the electrolytic water can be discharged to the stock unit 142B or the drain tank 157 in accordance with the operation condition of the air filtering apparatus 1. For example when crystal bodies, etc. are piled up in the water softening module 190, the crystal bodies can be directly discharged together with electrolytic water to the drain tank 157.

Still furthermore, the electrolytic water discharged from the water softening module 190 and the electrolytic water discharged from the electrolytic bath 146 are passed through the softened water return pipe 161 and the electrolytic water discharge pipe 173 and poured to the filter 174 respectively, passed through the filter 174 while scales, crystal bodies, etc. are removed from the electrolytic water, and then returned to the stock unit 142B. Therefore, even when scales, crystal bodies, etc. occurring in the water softening module 190 and the electrolytic bath 146 flow together with electrolytic water, they are collected by the filter 174, so that the scales, the crystal bodies, etc. can be prevented from flowing and circulating through the electrolytic water circulation passage containing the gas-liquid contact member 153.

A large amount of crystal bodies are precipitated in the fabric body 196 which is electrically connected to the electrode 194, and thus the concentration of the hardness components in electrolytic water can be lowered. Therefore, deposition of calcium ion, magnesium ion, etc. contained in electrolytic water occurs hardly at places other than the fabric body 196, and deposition of scales in the electrolytic water circulation passage containing the gas-liquid contact member 153 can be suppressed.

Furthermore, by executing the water softening operation mode, the hardness components such as calcium, magnesium, etc. contained in electrolytic water can be precipitated as crystal bodies and collected in the fabric body 196, whereby the hardness of the electrolytic water can be lowered.

Still furthermore, by executing the cleaning operation mode, the crystal bodies precipitated in the fabric body 196 of the water softening module 190 can be dissolved and exfoliated and removed from the water softening module 190. Accordingly, the water softening function of the water softening module 190 can be kept under an excellent state, and the hardness of electrolytic water can be lowered, so that deposition of scales can be efficiently suppressed.

The electrolytic water containing the dissolved and exfoliated crystal bodies occurring in the cleaning operation mode is discharged to the drain tank 157, so that the drainage in the cleaning operation mode which contains an amount of hardness components can be discharged to the outside of the electrolytic water circulation passage.

The second embodiment described above may be modified as follows.

For example, in the second embodiment, the voltage is applied to the electrodes 194 and 195 to precipitate crystal bodies in the fabric body 196 serving as the cathode, thereby softening electrolytic water. However, the second embodiment is not limited to the above style. For example, a water softening module having ion exchange resin may be used. Furthermore, the cleaning operation mode is executed by the controller 37 every time the accumulated actuation time of the water softening operation mode reaches five hours. However, the cleaning operation mode may be executed on the basis of an instruction from a user or the like, or it may be executed while the air filtering operation is stopped. Tap water serving as a raw material of electrolytic water is supplied from the water supply tank 141. However, the air filtering apparatus 101 may be connected to a water supply source of tap water or the like so as to be supplied with water.

Furthermore, in the electrolytic bath 146, ozone ($O_3$) or hydrogen peroxide ($H_2O_2$) may be generated as active oxygen species. In this case, when a platinum tantalum electrode is used as an electrode, active oxygen species can be stably generated with high efficiency by electrolyzing even water having rare ion species.

At this time, at the anode, the reactions represented by the following formulas occur and ozone is generated.

$$2H_2O \rightarrow 4H^+ + O_2 + 4e^-$$

$$3H_2O \rightarrow O_3 + 6H^+ + 6e^-$$

$$2H_2O \rightarrow O_3 + 4H^+ + 4e^-$$

At the cathode, the reactions represented by the following formulas, and $O_2^-$ generated through the electrode reaction is bonded with $H^+$ in solution to generate hydrogen peroxide ($H_2O=2$)

$$4H^+ + 4e^- + (4OH^-) \rightarrow 2H_2 + (4OH^-)$$

$$O_2^- + e^- + 2H^+ \rightarrow H_2O_2$$

Furthermore, in the second embodiment, tap water is supplied from the water supply tank 141. Tap water is added with chlorine compound for the purpose of sterilization, and thus chloride ion is contained. Accordingly, this chloride ion reacts to generate hypochlorous acid and hydrochloric acid. This is not limited to the case where tap water is used, but active oxygen species containing halogen is generated by the same reaction insofar as water supplied to the electrolytic bath 46 contains halide ion because halide is added or contaminated.

In the air filtering apparatus 101, the same reaction can be induced even when water having rare ion species (containing pure water, purified water, well water, some tap water, etc.) is used. That is, by adding halide (brine or the like) to water having rare ion species, the same reaction occurs, and active oxygen species can be obtained. It is needless to say that the detailed construction of the air filtering apparatus 101 may be arbitrarily changed.

Third Embodiment (Not according to the claimed invention)

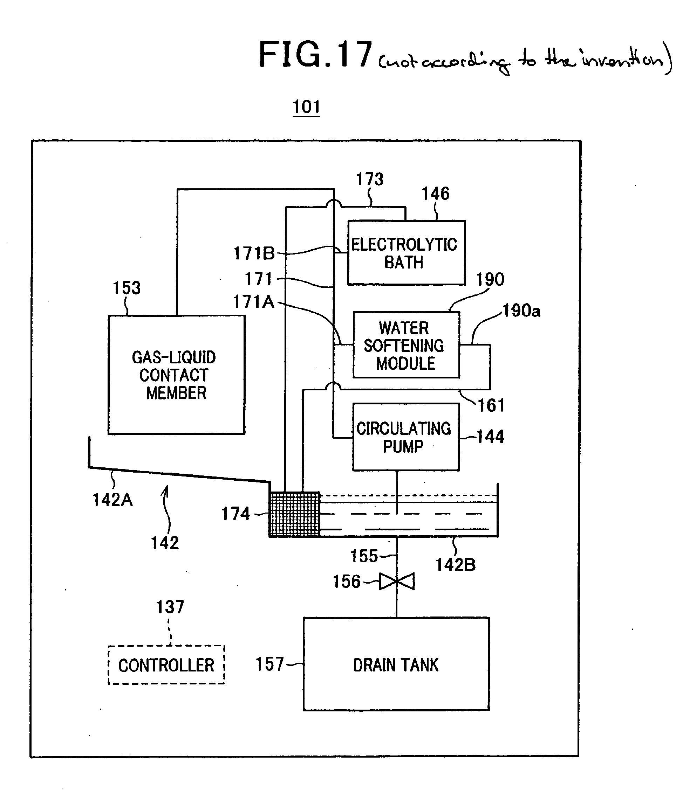

A third embodiment will be described hereunder with reference to Fig. 17. In the third embodiment, the same parts as the second embodiment are represented by the same reference numerals, and the description thereof is omitted.

The third embodiment is different from the second embodiment in that the electrolytic water discharged from the water softening module 190 does not flow to the drain tank 157 and all the electrolytic water flows to the stock unit 142B.

Fig. 17 is a schematic diagram showing an electrolytic water circulation passage according to the third embodiment.

A softened water return pipe 161 for returning softened electrolytic water to the stock unit 142B is connected to the discharge port 190a of the water softening module 190. The terminal of the water softening return pipe 161 is located above the filter 174, and electrolytic water discharged from the water softening module 190 is directly poured from the terminal of the softened water return pipe 161 to the filter 174 and then returned to the stock unit 142B.

That is, the crystal bodies exfoliated in the cleaning operation mode are passed through the softened water return pipe 161 together with the electrolytic water, and reaches the filter 174 to be collected.

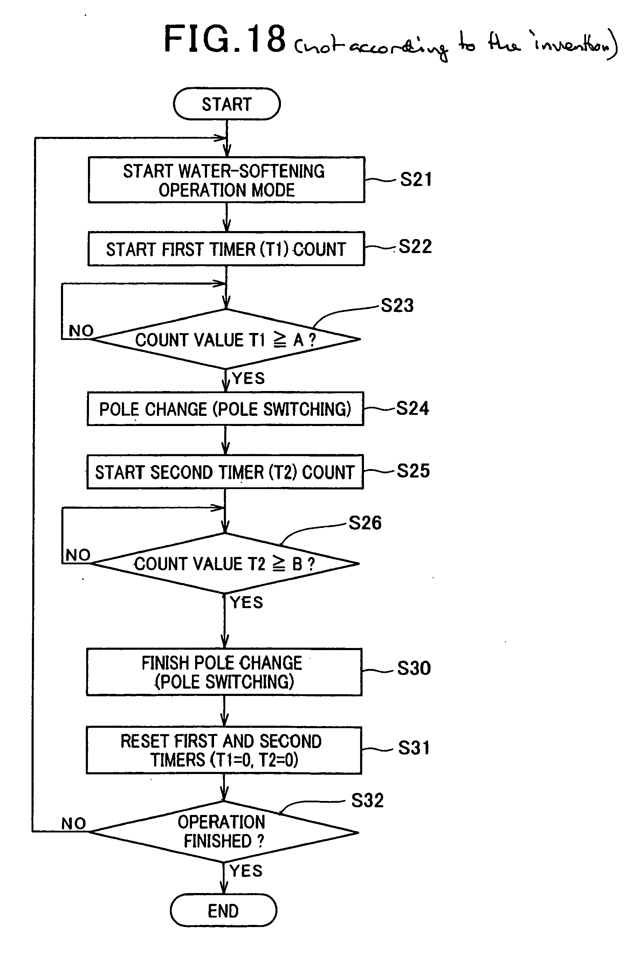

Fig. 18 is a flowchart showing the processing of the cleaning operation mode.

The steps of executing the same processing of Fig. 16 in the second embodiment out of the processing contained in the operation shown in Fig. 18 are represented by the same reference numerals.

The controller 137 starts the water softening operation mode along with the start of the air filtering operation (step S1), and starts the count of the first timer for counting the accumulated actuation time of the water softening operation mode (step S2). The controller 137 refers to the count value T1 of the first timer and executes the water softening operation mode until the timer count value T1 reaches the set value A (step S3).

When the count value T1 of the first timer reaches the set value A (step S3: Yes), the controller 137 switches the polarities of the electrodes 194 and 195 to the opposite polarities to those in the water softening operation mode and the applies a voltage (change the polarities) between the electrodes (step S4), starts the count of the second timer (step S5), and starts the cleaning operation mode.

The controller 37 continues the cleaning operation mode until the count value T2 of the second timer reaches the set value B (step S6), and stops the change of the polarities of the electrodes 194 and 195 when the count value T2 reaches the set value B (step S6: Yes), whereby the polarities of the electrodes 194 and 195 are set to the same polarities in the water softening operation mode (step S10). Thereafter, the controller 137 resets the first timer and the second timer (step S11), and determines whether the operation is finished or not (step S12). When the operation is continued, the processing returns to step S1. When it is instructed to finish the air filtering operation or the water softening operation mode (step S12; Yes), the operation of Fig. 18 is finished.

The air filtering operation is started at the start time of the operation of Fig. 18, so that the circulating pump 144 is actuated and electrolytic water is continuously supplied to the water softening module 190 by the circulating pump 144. Therefore, the operation of Fig. 18 is carried out while the actuation state of the circulating pump 144 is maintained, the change of poles is carried out in step S5 and the voltage is applied under the pole-changed state until the count value T2 reaches the set value B, whereby the crystal bodies precipitated in the water softening module 190 are discharged from the water softening module 190 and the function of the water softening module 190 is kept under an excellent state.

In the third embodiment, electrolytic water discharged form the water softening pipe 161 is passed through the filter 174 and reaches the stock unit 142B. The crystal bodies dissolved and exfoliated in the cleaning operation mode flows through the water softening return pipe 161 together with electrolytic water, and thus the crystal bodies discharged from the water softening module 190 can be collected by the filter 174.

According to this construction, the construction of collecting the crystal bodies exfoliated in the cleaning operation mode can be simply implemented without providing the thee-way valve 162 and the drain pipe 163 of the second embodiment. In this case, the dissolved hardness components are contained in the electrolytic water discharged from the water softening module 190, and thus it is desired to discharge the electrolytic water of the stock portion 142B to the drain tank 157 by opening the drain valve 156. When the cleaning operation mode is finished, the controller 137 may open the drain valve 156 for a predetermined time to discharge the electrolytic water in the stock unit 142B to the drain tank 157.

Furthermore, the circulating pump 144 is also actuated during the cleaning operation mod and thus water flow occurs in the water softening module 190. Therefore, the exfoliation of the crystal bodies is further promoted by this water flow.

The third embodiment described above is an embodiment to which various modifications may be made. For example, in the third embodiment, the cleaning operation mode is executed in parallel to the air filtering operation, and the circulating pump 144 continues to operation during even the cleaning operation mode. However, this embodiment may be modified so that the cleaning operation mode is started under the state that the air filtering operation is stopped and the circulating pump 144 is stopped, and the poles of the electrodes are reversed to dissolve and exfoliate crystal bodies, and then the circulating pump 144 is actuated. The other detailed constructions may be arbitrarily changed.


Fourth Embodiment (Not according to the invention as claimed)


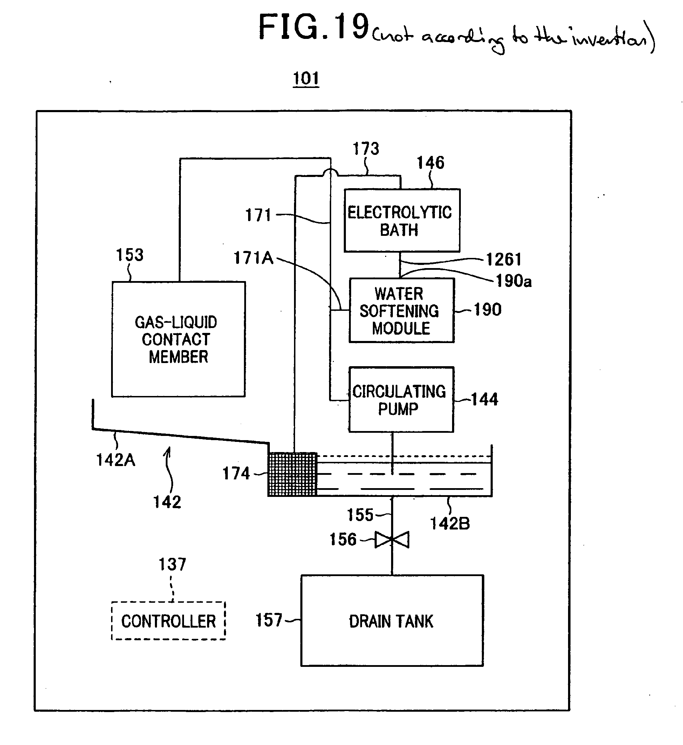

A fourth embodiment will be described with reference to Fig. 19.

In the fourth embodiment, the same constituent elements as the third embodiment are represented by the same reference numerals.

The fourth embodiment is different from the third embodiment in that electrolytic water softened in the water softening module 190 is directly supplied to the electrolytic bath 146.

Fig. 19 is a schematic diagram showing an electrolytic water circulation passage according to the fourth embodiment.

A discharge pipe 1261 connected to the flow-in port of electrolytic water in the electrolytic bath 146 is connected to the discharge port 190a of the water softening module 190. The second branch pipe 171B used in the second and third embodiments is not connected to the electrolytic bath 146. Electrolytic water which is pumped up by the stock unit 142B and softened in the water softening unit module 190 is directly fed to the electrolytic bath 146 to be electrolyzed, and the electrolytic water is passed through the electrolytic water discharge pipe 173 and the filter 174 and then returned to the stock unit 142B.

The terminal of the electrolytic water discharge pipe 173 is located above the filter 174, and the electrolytic water which is passed through the water softening module 190 and discharged from the electrolytic bath 146 reflows so as to be directly poured to the filter 174, and then it is returned to the stock unit 142B>

That is, scales generated in the electrolytic bath 146 and crystal bodies exfoliated in the water softening module 190 are passed through the electrolytic water discharge pipe 173 together with electrolytic water and reaches the filter 174, so that the scales and the crystal bodies are caught and collected by the filter 174.

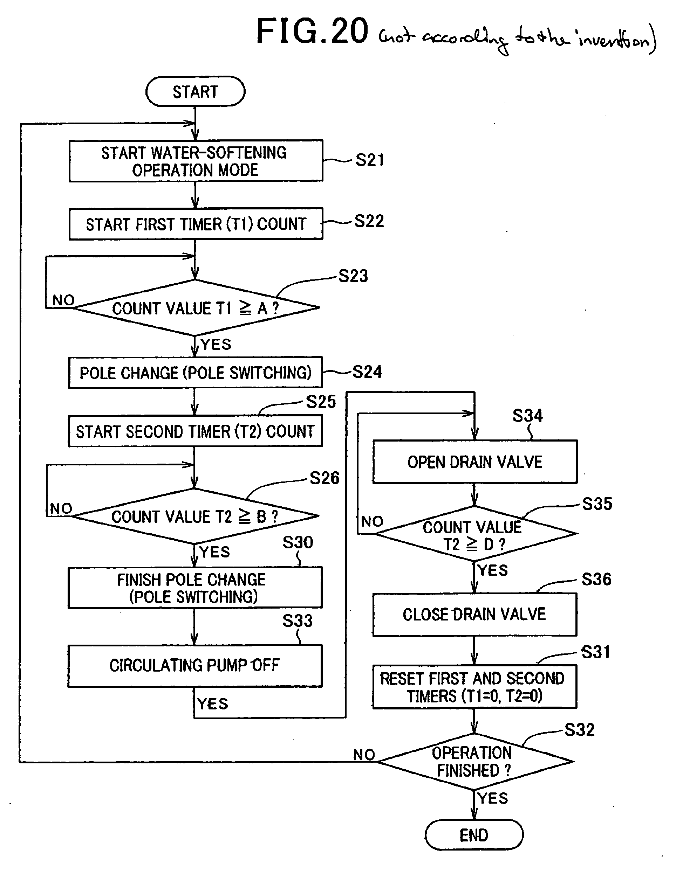

Fig. 20 is a flowchart showing the operation of the cleaning operation mode according to the fourth embodiment.

The steps of executing the same processing as the second embodiment are represented by the same reference numerals.

The controller 137 starts the water softening operation mode together with the start of the air filtering operation (step S1), and starts the count of the first timer (step S2). The controller 137 refers to the count value T1 of the first timer, and executes the water softening operation mode until the count value T1 of the timer reaches the set value A (step S3).

The controller 137 starts the water softening operation mode together with the start of the air filtering operation (step S1), and starts the count of the first timer for counting the accumulated actuation time of the water softening operation mode (step S2). The controller 137 refers to the count value T1 of the first timer, and executes the water softening operation mode until the count value T1 of the timer reaches the set value A (step S3).

When the count value T1 of the first timer reaches the set value A (step S3: Yes), the controller 137 switches the polarities of the electrodes 194 and 195 to the opposite polarities to those in the water softening operation mode and then the voltage is applied (pole change) (step 5), the count of the second timer is started (step S6), and the cleaning operation mode is started.

The controller 137 continues the cleaning operation mode until the count value T2 of the second timer reaches the set value B (step S6), and when the count value T2 reaches the set value B (step S6: Yes), the controller 137 finishes the pole change of the electrodes 194 and 195, returns the polarities to the same polarities in the water softening operation mode (step S10). In step S10, the controller 137 may not only finish the pole change of the water softening module 190, but also stop the actuation of the water softening module 190.

The circulating pump 144 is actuated during the cleaning operation mode which is executed from step S5 till step S10, and thus crystal bodies dissolved and exfoliated in the water softening module 190 are discharged to the stock unit 142B together with electrolytic water.

Subsequently, the controller 137 stops the circulating pump 144 (step S21), and opens the drain valve 156 (step S22). The controller 137 sets the drain valve 156 to an open state until the count value T2 of the second timer reaches a preset set value D (step S23). When the count value T2 reaches the set value D (step S23: Yes), the drain valve 156 is closed (step S24). Here, the set value D is a value for determining a time for discharging electrolytic water to the drain tank 157 by opening the drain valve in step S22, and for example it is preferable that most of the electrolytic water of the stock unit 142B can be discharged. The discharge time of the electrolytic water corresponds to the time period from the time when the count value T2 of the second timer reaches the set value B till the time when the count value T2 reaches the set value D, and thus the set value D corresponds to the sum of the discharge time of the discharge to the drain tank 157 and the set value B. For example, when the set value B is set to the value corresponding to 10 minutes of the actual time, in order to discharge electrolytic water from the stock unit 142B for 1 minute, the set value is set to the count value corresponding to 11 minutes (actual time) obtained by summing 10 minutes and 1 minute. The operation time of step S21 is very shorter than the discharge time of one minute, and thus the discharge time of one minute is set while this operation is contained in the discharge time.

After the electrolytic water is discharged to the drain tank 157, the controller 137 resets the firs timer and the second timer (step S11), and determines whether the operation should be finished or not (step S12). When the operation is continued, the processing returns to step S1. When it is instructed to finish the air filtering operation or the water softening operation mode (step S12: Yes), the operation of Fig. 20 is finished.

As described above, in the operation shown in Fig. 20, the operation of the three-way valve 162 out of the operation of Fig. 8 described above is omitted, however, the opening/closing operation of the drain valve 156 is added.

According to the operation of Fig. 20, deposits such as crystal bodies, etc. adhering to the inside of the water softening module 190 can be removed, and deposits such as crystal bodies dissolved and exfoliated in the cleaning operation mode are discharged to the stock unit 142B together with electrolytic water. Here, solid deposits are collected by the filter 174, and electrolytic water containing dissolved crystal bodies is discharged from the stock unit 142B to the drain tank 157. Accordingly, the deposits such as crystal bodies, etc. adhering to the water softening module 190 can be easily removed, and the function of the air filtering apparatus 1 can be kept under an excellent state.

In the fourth embodiment, the water softening module 190 and the electrolytic bath 146 are directly connected to each other through the discharge pipe 1261, so that electrolytic water softened in the water softening module 190 is directly supplied to the electrolytic bath 146. Therefore, electrolytic water to be electrolyzed in the electrolytic bath 146 is softened and thus the hardness thereof is lowered. Therefore, adhesion of deposits such as scales, etc. on the electrodes 47 and 48 of the electrolytic bath 146 can be suppressed.

Furthermore, the water softening module 190 and the electrolytic bath 146 are directly connected to each other, and the softening of electrolytic water and electrolysis are performed in one pipe passage, so that the pipe construction can be simply implemented.

Fifth Embodiment (Not according to the invention as claimed)

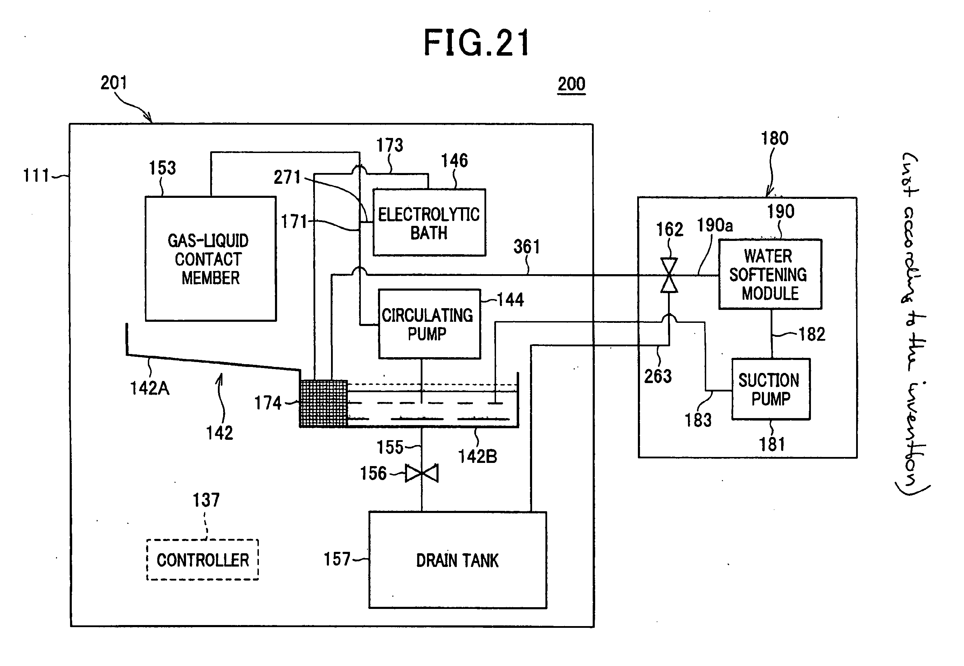

A fifth embodiment will be described hereunder with reference to Fig. 21.

In the fifth embodiment, the same constituent elements as the second embodiment are represented by the same reference numerals, and the description thereof is omitted. The fifth embodiment is different from the second embodiment in that a water softening unit 280 constructed as a separate body from the air filtering apparatus main body 201 is externally equipped in place of the water softening module 190 of the second embodiment.

Fig. 21 is a schematic diagram showing an electrolytic water circulation passage according to the fifth embodiment.

An air filtering apparatus 200 has an air filtering apparatus main body 201 including the gas-liquid contact member 153, the circulating pump 144, the electrolytic bath 146, the water receiving tray 142, etc., and the water softening unit 180 constructed as a separate body from the air filtering apparatus main body 201, which are mounted in the housing 111.

Electrolytic water is circulated in the air filtering apparatus main body 201 as follows.

The electrolytic water in the stock unit 142B is pumped up by the circulating pump 144, passed through the water distribution pipe 171 and supplied to the gas-liquid contact member 153. Thereafter, electrolytic water dropping from the gas-liquid contact member 153 is returned to the stock unit 142B. The branch pipe 171 branched from the water distribution pipe 171 is connected to the water distribution pipe 171, and electrolytic water flowing to the branch pipe 171 is passed through the electrolytic bath 146 and the electrolytic water discharge pipe 173, and then returned to the stock unit 142B through the filter 174. As described above, in the air filtering apparatus main body 201, electrolytic water is supplied from the stock unit 142B to the gas-liquid contact member 153 and the electrolytic bath 146 and then returned to the stock unit 142B again.

In the water softening unit 180 are disposed a water softening pump 181 for pumping up electrolytic water in the stock unit 142B and a water softening module 190 for softening electrolytic water sucked by the suction pump 181. Here, the water softening module 190 has the same construction as the water softening module 190 of the first embodiment. The respective parts of the water softening unit 180 are connected to the controller 137 of the air filtering apparatus main body 201.

A suction pipe 183 for connecting the stock unit 142B and the suction port of the suction pump 181 is connected to the suction port of the suction pump 181. A discharge pipe 182 for supplying electrolytic water sucked by the suction pump 181 to the water softening module 190 is connected to the discharge port of the suction pump 181. A three-way valve 162 is connected to the discharge port 190a for discharging electrolytic water softened in the water softening module 190, and a softened water return pipe 361 for returning softened electrolytic water to the stock unit 142B is connected to one outlet of the three-way valve 162, and a drain pipe 263 connected to the drain tank 157 is connected to the other outlet of the three-way valve 162. The terminal of the softened water return pipe 361 is located above the filter 174, and electrolytic water discharged from the water softening module 190 is directly poured from the terminal of the softened water return pipe 361 to the filter 174, and returned to the stock unit 142B. The three-way valve 162 is switched on the basis of an instruction of the controller 137.

The suction pipe 183, the softened water return pipe 361 and the drain pipe 263 extend to the outside of the water softening unit 180, and they are disposed so as to bridge the air filtering apparatus main body 201 and the water softening unit 180. In detail, each of the suction pipe 183 and the softened water return pipe 361 connects the water softening unit 180 and the stock unit 142B, and the drain pipe 263 connects the water softening unit 180 and the drain tank 157.

The circulation of the electrolytic water flowing in the water softening unit 180 is performed as follows.

First, electrolytic water in the stock unit 142B is sucked from the suction pipe 183 by the suction pump 181, passed through the discharge pipe 182 and then reaches the water softening module 190. Electrolytic water discharged from the discharge port 190a of the water softening module 190 is passed through the three-way valve 162, passed through the softened water return pipe 361 and returned to the stock unit 142B again. As described above, in the water softening unit 180, electrolytic water is circulated so as to be supplied from the stock unit 142B to the water softening module 190 to be softened and then returned to the stock unit 142B. Furthermore, electrolytic water softened by the water softening module 190 can be directly discharged to the drain tank 157 by switching the three-way valve 162 under the control of the controller 137.

That is, the water softening unit 180 is constructed as a separate body from the air filtering apparatus main body 201, and also it has the suction pump 181 so that electrolytic water can be sucked by itself. Furthermore, electrolytic water circulated in the water softening unit 180 is circulated in the air filtering apparatus main body 201 independently of the circulation of the electrolytic water in the air filtering apparatus main body 201. Therefore, the suction pipe 183, the softened water return pipe 361 and the drain pipe 263 are constructed so as to bridge the water softening unit 180 and the air filtering apparatus main body 101. Therefore, the water softening unit 180 and the air filtering apparatus main body 201 can be easily externally provided.

When the air filtering apparatus 200 is instructed to start the air filtering operation, the controller 137 actuates the fan motor 132, the circulating pump 144, the electrolytic bath 146, etc. of the air filtering apparatus main body 201, and actuates the air filtering operation of circulating electrolytic water in the circulation passage containing the gas-liquid contact member 153. In connection with this operation, the controller 137 actuates the suction pump 181 of the water softening unit 180 and the water softening module 190, and executes the water softening operation mode. Furthermore, the controller 137 executes the cleaning operation mode every time the accumulated actuation time of the water softening operation mode reaches a predetermined time.

Fig. 14 is a flowchart showing the processing of the cleaning operation mode.

In the processing contained in the operation shown in Fig. 14, the steps executing the same processing as shown in Fig. 16 in the first embodiment are represented by the same reference numerals.

When starting the water softening operation mode together with the start of the air filtering operation (step S1), the controller 137 starts the count of the first timer (step S2). Thereafter, the controller 137 refers to the count value T1 of the first timer, and executes the water softening operation mode until the count value T1 of the timer reaches a preset set value A (step S3).

When the count value T1 of the first timer reaches the set value A (step S3: Yes), the controller 137 switches the polarities of the electrodes 194 and 195 to the opposite polarities to those in the water softening operation mod and then applies the voltage (pole change) (step S4), starts the count of the second timer (step S5), and starts the cleaning operation mode. In this cleaning operation mode, the polarities of the electrodes 194 and 195 are reversed, and crystal bodies precipitated in the water softening module 190 are dissolved and exfoliated.

Furthermore, the suction pump 181 is actuated during even the cleaning operation mode, and thus the crystal bodies dissolved and exfoliated in the water softening module 190 flow to the stock unit 142B together with electrolytic water, and collected by the filter 174.

The controller 137 continues the cleaning operation mode until the count value T2 of the second timer reaches the set value B (step S6), and when the count value T2 reaches the set value B (step S6: Yes), the controller 137 switches the three-way valve 162 so that electrolytic water flows from the water softening module 190 to the drain pipe 263 (step S31), and starts discharge of the electrolytic water to the drain tank 157. Through the operation of the step S31, crystal bodies dissolved and exfoliated in the water softening module 190 are discharged to the drain tank 157 together with electrolytic water.

Here, the set value B is set in the same manner shown in Fig. 16. However, in the fifth embodiment (Fig. 20), the set value B is treated as a value for determining a time until the three-way valve 162 is switched and the discharge of electrolytic water to the drain tank 157 is started. The second timer continues the count after the count value T2 reaches the set value B.

Subsequently, the controller 137 is on standby until the count value T2 of the second timer reaches the set value C under the state that the electrolytic water in the water softening module 190 is discharged to the drain tank 157 (step S8). Here, when the count value T2 reaches the set value C (step S8: Yes), the controller 137 returns the switching state of the three-way valve 162 to the original state so that electrolytic water flows to the softened water return pipe 361 again (step S32), finishes the pole change of the electrodes 194 and 195, and returns the polarities of the electrodes to the same polarities in the water softening operation mode (step S10).

The set value C is set as in the same manner as shown in Fig. 16. However, in the fifth embodiment (Fig. 20), it is treated as a value for determining a time for switching the three-way valve 162 and discharging electrolytic water to the drain tank 157.

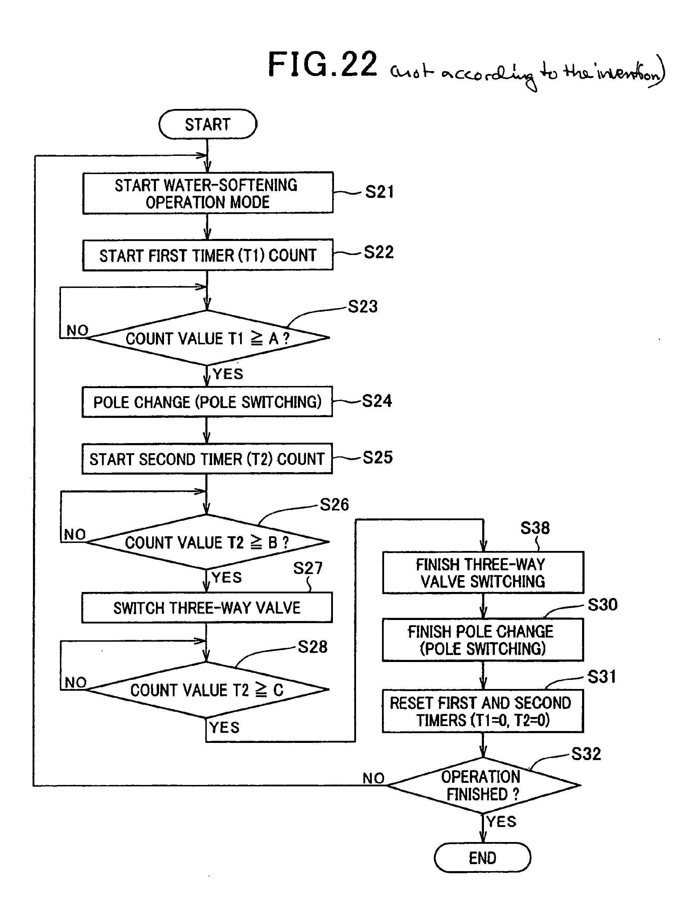

After the switching state of the three-way valve 162 and the pole-change state of the electrodes 194 and 195 are returned to the original states, the controller 137 resets the first timer and the second timer (step S11), and determines whether the operation is finished or not (step S12). When the operation is continued, the processing returns to step S1. When it is instructed to finish the air filtering operation and the water softening operation mode (step S12: Yes), the operation of Fig. 22 is finished.

As described above, in the fifth embodiment, the externally-equipped water softening unit 180 has the suction pump 181 different from the circulating pump 144, and switches the three-way valve 162 to discharge electrolytic water to the drain tank 157. Furthermore, the circulating pump 144 can be actuated independently of the operation of Fig. 22.

Through the operation of Fig. 22, crystal bodies precipitated on the surface of the fabric body 196 of the water softening module 190 are dissolved and exfoliated, and further drifted by the water flow under the actuation of the suction pump 181 to promote the exfoliation, so that the crystal bodies are discharged to the drain tank 157 together with electrolytic water.

According to the fifth embodiment, the water softening unit 180 can be externally equipped to the air filtering apparatus main body 201, and thus the water softening unit 180 can be afterwards equipped as occasion demands. For example, the water softening unit 180 may be provided as an option of the air filtering apparatus 200, and the water softening function can be added or deleted in accordance with user's requirement. Furthermore, the water softening unit 180 may be externally equipped to an existing air filtering apparatus to add the water softening function.

Furthermore, the water softening unit 180 has the suction pump 181 independently of the circulating pump 144. Therefore, the cleaning operation mode can be independently executed. Furthermore, electrolytic water discharged in the cleaning operation mode can be discharged to the drain tank 157. Therefore, drain of the cleaning operation mode containing lots of hardness components can be discharged to the outside of the electrolytic water circulation passage. Therefore, the electrolytic water of the stock unit 142B can be kept under a state which is proper to the air filtering operation.

The fifth embodiment is an embodiment to which various modifications may be made.

In the fifth embodiment, the electrolytic water discharged in the cleaning operation mode is discharged to the drain tank 157. The fifth embodiment is not limited to this style. The fifth embodiment may be modified so that the three-way valve 162 and the drain pipe 263 are not provided, and the electrolytic water is discharged to the stock unit 142B by the softened water return pipe 361. The other detailed constructions may be arbitrarily changed.

The present invention is defined in the claims and otherwise not limited to the above-described embodiments. Various modifications and alterations may be made on the basis of the technique idea of the present invention.

For example, in the above embodiments, the air filtering apparatus 1 is supplied with tap water. However, water having rare ion species (containing purified water, well water or the like, for example) other than tap water may be supplied to the air filtering apparatus 1. In this case, a sufficient air filtering (cleaning) effect (inactivation of virus, etc., sterilization, deodorization, etc.) can be exercised by supplying brine from the bring tank 90.

Furthermore, in the above embodiments, the water supply nozzle 94 is disposed so as to be far away from the water level of electrolytic water stocked in the water receiving tray 42. However, the water supply nozzle 94 may be immersed in the electrolytic water stocked in the water receiving tray 42. In this case, the check valve 94A may be formed of a material having resistance to electrolytic water (for example, Hastelloy (trademark)). In this construction, the water supply nozzle 94 is provided with the check valve 94A, and thus even when the internal pressure of the tubes 92, 93 and the brine tank 90 is negative, water stocked in the water receiving tray 42 can be prevented from flowing back through the water supply nozzle 94 into the tubes 92, 93 and the brine tank 90.

Still furthermore, in the invention as claimed, brine which can be handled safely and at low price is stocked in the brine tank 90. However, the other embodiment are not limited to brine, and water containing halide ion such as chlorine ion or the like may be stocked. In this case, active oxygen species containing halogen is generated through the same reaction as the formulas (3) and (4).

In the above embodiments, tap water (drinking water) is supplied through the water supply pipe 27 to the water receiving tray 42. However, a water supply tank 41 which stocks water and can be freely taken into/out of the air filtering apparatus 1 may be provided so that water is supplied from the water supply tank to the water receiving tray 42.

Furthermore, in the above embodiments, the air filtering apparatus 1 is supplied with tap water having a low chlorine concentration. However, water having rare ion species (containing pure water, purified water, well water or the like) other than tap water may be supplied. In this case, brine is supplied from the brine tank 90, whereby a sufficient air filtering (cleaning) effect (inactivation of virus, etc., sterilization, deodorization, etc.) can be exercised.

**Claims**

1. An air filtering apparatus comprising
   a gas-liquid contact member;
   an electrolytic unit adapted to generate electrolytic water;
   supply means for supplying the electrolytic water to the gas-liquid contact member to infiltrate the electrolytic water into the gas-liquid contact member and to reflow to a water receiving tray;
   an air blowing fan for feeding air to the gas-liquid contact member to bring the electrolytic water into contact with the air in the gas-liquid contact member to filter the air;
   a brine supply unit having a brine tank stocking brine and being adapted to supply brine to the water receiving tray; and
   a controller being adapted to set a water exchange operation mode in which water stocked in the water receiving tray is discharged and new water is supplied into the water receiving tray and to operate the brine supply unit when the water exchange operation mode is selected and to supply brine into the water receiving tray;
   **characterized in that** the controller has an electric conductivity detecting unit adapted to detect the electric conductivity of water reflowing to the water receiving tray and that the controller is adapted to supply brine to the water receiving tray in accordance with the electric conductivity of the water.

2. The air filtering apparatus according to claim 1, wherein the controller is adapted to repeat discharge of water stocked in the water receiving tray and supply of new water until the electric conductivity of water reflowing in the water receiving tray is equal to a predetermined value or less when the water exchange operation is carried out, and to operate the brine supply unit to supply brine into the water receiving tray when the electric conductivity of the water reaches the predetermined value or less.

3. The air filtering apparatus according to claim 1, wherein the controller has an air filtering operation mode for filtering air and the controller is adapted to stop the air filtering operation mode every predetermined time, and to resume the air filtering operation mode when the water exchange operation mode is selected.

**Patentansprüche**

1. Luftfiltergerät, das Folgendes umfasst:

   ein Gas-Flüssigkeit-Kontaktelement;
   eine Elektrolyteinheit, die eingerichtet ist, elektrolytisches Wasser zu erzeugen;
   Zufuhrmittel zum Zuführen des elektrolytischen Wassers zu dem Gas-Flüssigkeit-Kontaktelement, um das elektrolytische Wasser in das Gas-Flüssigkeit-Kontaktelement eindringen zu lassen und in eine Wasseraufnahmewanne zurückfließen zu lassen;
   einen Luftgebläseventilator zum Zuführen von Luft zu dem Gas-Flüssigkeit-Kontaktelement, um das elektrolytische Wasser mit der Luft in dem Gas-Flüssigkeit-Kontaktelement in Kontakt zu bringen, um die Luft zu filtrieren;
   eine Salzlösungszufuhreinheit, die einen Salzlösungsbehälter aufweist, der Salzlösung lagert und eingerichtet ist, Salzlösung der Wasseraufnahmeschale zuzuführen; und
   eine Steuerung, die eingerichtet ist, eine Wasseraustauschbetriebsart festzusetzen, bei welcher Wasser, das in der Wasseraufnahmewanne gelagert ist, abgelassen wird, und neues Wasser in die Wasseraufnahmewanne zugeführt wird, und die Salzlösungszufuhreinheit zu betreiben, wenn die Wasseraustauschbetriebsart ausgewählt ist, und Salzlösung in die Wasseraufnahmewanne zuzuführen;
**dadurch gekennzeichnet, dass** die Steuerung eine Erfassungseinheit für elektrische Leitfähigkeit aufweist, die eingerichtet ist, die elektrische Leitfähigkeit von Wasser zu erfassen, das zu der Wasseraufnahmewanne zurückfließt, und dass die Steuerung eingerichtet ist, Salzlösung zu der Wasseraufnahmewanne in Übereinstimmung mit der elektrischen Leitfähigkeit des Wassers zuzuführen.

2. Luftfiltergerät gemäß Anspruch 1, wobei die Steuerung eingerichtet ist, den Auslass von Wasser, das in der Wasseraufnahmewanne gelagert wird, und die Zufuhr von neuem Wasser zu wiederholen, bis die elektrische Leitfähigkeit von Wasser, das in die Wasseraufnahmewanne zurückfließt, gleich einem vorgegebenen Wert oder weniger ist, wenn der Wasseraustauschbetrieb durchgeführt wird, und die Salzlösungszufuhreinheit zum Zuführen von Salzlösung in die Wasseraufnahmewanne zu betreiben, wenn die elektrische Leitfähigkeit des Wassers den vorgegebenen Wert oder weniger erreicht.

3. Luftfiltergerät gemäß Anspruch 1, wobei die Steuerung eine Luftfilterbetriebsart zum Filtrieren von Luft aufweist und die Steuerung eingerichtet ist, die Luftfilterbetriebsart jedes vorgegebene Mal anzuhalten und die Luftfilterbetriebsart wieder aufzunehmen, wenn die Wasseraustauschbetriebsart ausgewählt ist.

## Revendications

1. Dispositif de filtrage d'air comprenant :

un élément de contact gaz-liquide ;
une unité électrolytique adaptée afin de produire de l'eau électrolytique ;
un moyen d'alimentation destiné à délivrer l'eau électrolytique à l'élément de contact gaz-liquide afin d'infiltrer l'eau électrolytique dans l'élément de contact gaz-liquide et de la mettre en recirculation vers un plateau de réception d'eau ;
un ventilateur de soufflage d'air destiné à délivrer de l'air à l'élément de contact gaz-liquide afin d'amener l'eau électrolytique en contact avec l'air sur l'élément de contact gaz-liquide de manière à filtrer l'air ;
une unité d'alimentation en saumure comportant un réservoir de saumure stockant de la saumure et adaptée afin de délivrer de la saumure au plateau de réception d'eau ; et
une unité de commande qui est adaptée afin de positionner un mode de commande d'échange d'eau dans lequel l'eau stockée dans le plateau de réception d'eau est évacuée et de l'eau nouvelle est délivrée au plateau de réception d'eau et afin d'activer l'unité d'alimentation en saumure lorsque le mode de commande d'échange d'eau est sélectionné et de délivrer de la saumure au plateau de réception d'eau ;
**caractérisé en ce que** l'unité de commande comporte une unité de détection de conductivité électrique adaptée afin de détecter la conductivité électrique de l'eau en recirculation dans le plateau de réception d'eau et **en ce que** l'unité de commande est adaptée afin de délivrer de la saumure au plateau de réception d'eau en fonction de la conductivité électrique de l'eau.

2. Dispositif de filtrage d'air selon la revendication 1, dans lequel l'unité de commande est adaptée afin de répéter l'évacuation de l'eau stockée dans le plateau de réception d'eau et de délivrer de l'eau nouvelle jusqu'à ce que la conductivité électrique de l'eau en recirculation dans le plateau de réception d'eau devient inférieure ou égale à une valeur prédéterminée lorsque l'opération d'échange d'eau est mise en oeuvre et afin de commander l'unité d'alimentation en saumure de manière à délivrer de la saumure au plateau de réception d'eau lorsque la conductivité électrique de l'eau atteint une valeur prédéterminée ou inférieure.

3. Dispositif de filtrage d'air selon la revendication 1, dans lequel l'unité de commande présente un mode de commande de filtrage d'air destiné à filtrer l'air et l'unité de commande est adaptée de manière à arrêter le mode de commande de filtrage d'air au bout de chaque période prédéterminée, et à reprendre le mode de commande de filtrage d'air lorsque le mode de commande d'échange d'eau est sélectionné.

# FIG.1

# FIG.2

# FIG.3

# FIG.4

# FIG.5

# FIG.6

# FIG.7A

# FIG.7B

# FIG.8

POWER SUPPLY UNIT 109

DRAIN VALVE 83

FAN MOTOR 107

LOUVER DRIVING MOTOR 108

CIRCULATING PUMP 44

100

TIMER COUNT 103

MICROCOMPUTER 101M

INPUT UNIT 104

STORAGE UNIT 102

OUTPUT UNIT 105

PUMP 95

ELECTRIC CONDUCTIVITY METER 49

ELECTRODE 47,48

ELECTROLYTIC BATH FS 110

WATER RECEIVING TRAY FS 43A,43B

OPERATING PANEL 106

EP 2 145 631 B1

# FIG.9

```
( BRINE SUPPLY OPERATION )
            │
            ▼
    ┌──────────────────┐
    │ INITIALIZE COUNTER │──── S1
    └──────────────────┘
            │
            ▼
 ┌──────────────────────────────┐
 │ DETECT ELECTRIC CONDUCTIVITY D1 │──── S2
 └──────────────────────────────┘
            │
            ▼                                    S3
    ◇ ELECTRIC CONDUCTIVITY                      NO
      D1 ≦ PREDETERMINED ELECTRIC  ──────────────────┐
      CONDUCTIVITY Dm1 ? ◇                            │
            │                                         ▼                  S4
           YES                         ◇ WATER EXCHANGE                  NO          S5
            │                            OPERATION FREQUENCY ────────────────┐
            │                            COUNTER N1 ≧ 5 ? ◇                   ▼
            │                                  │                      ┌─────────────────┐
            │                                 YES                     │ WATER EXCHANGE  │
    S9      │                     S8  ┌──────────────────┐            │   OPERATION     │
    ┌───────────────┐                │ TURN ON AND OFF  │            └─────────────────┘
    │ STORE ELECTRIC │                │ INDICATOR LAMP   │                   │        S6
    │ CONDUCTIVITY D1│                └──────────────────┘            ┌─────────────────┐
    └───────────────┘                         │                      │ WATER EXCHANGE  │
    S10     │                              ( END )                    │   OPERATION     │
    ┌───────────────┐                                                 │ FREQUENCY COUNTER│
    │  SUPPLY BRINE │◀─────────────────────────────────┐              │   N1 = N1 + 1   │
    └───────────────┘                                  │              └─────────────────┘
            │                                           │                     │
            │                                           │              ◇ TIME            S7
            │                                           │        NO    T1 ≧ PREDETERMINED
            │                                           └──────────── TIME Tm1 ? ◇
            │                                                                │
            │                                                               YES
            ▼                                                                │
            │◀──────────────────────────────────────────────────────────────┘
    S11     │                              ┌────────────────────────┐
    ◇ TIME                                 │ ELECTRIC CONDUCTIVITY   │
      T2 ≧ PREDETERMINED      NO           │ DETECTION FREQUENCY     │──── S14
      TIME Tm2 ? ◇ ──────────────────────▶│ COUNTER   N2 = N2 + 1   │
            │                              └────────────────────────┘
           YES                                       │                       S15
    S12 ┌───────────────┐            ◇ ELECTRIC CONDUCTIVITY              NO
    │ DETECT ELECTRIC │              DETECTION FREQUENCY ──────────────────┐
    │ CONDUCTIVITY D2 │              COUNTER N2 ≧ 3 ? ◇                     │
    └───────────────┘                        │                             │
    S13     │                               YES                            │
    ◇ ELECTRIC CONDUCTIVITY      ┌──────────────────────────┐              │
      D2 ≧ ELECTRIC CONDUCTIVITY │ BRINE SUPPLY FREQUENCY   │──── S16       │
      D1 + PREDETERMINED ELECTRIC│ COUNTER   N3 = N3 + 1    │              │
      CONDUCTIVITY Dm2 ? ◇ ──NO──└──────────────────────────┘              │
            │                               │                       S17     │
           YES                     ◇ BRINE SUPPLY                    NO      │
            │                        FREQUENCY COUNTER ─────────────────────┘
         ( END )                     N3 ≧ 8 ? ◇
                                              │
                                             YES
                                   ┌──────────────────┐
                                   │ TURN ON AND OFF  │──── S18
                                   │ INDICATOR LAMP   │
                                   └──────────────────┘
                                              │
                                           ( END )
```

# FIG.10 (not according to the invention)

# FIG.11 (not according to the invention)

# FIG.12 (not according to the invention)

<u>101</u>

FIG.13 (not according to the invention)

# FIG.14 (not according to the invention)

101

173  146

ELECTROLYTIC
BATH

171B
171
171A

153

GAS-LIQUID
CONTACT
MEMBER

190
190a
162

WATER
SOFTENING
MODULE

163

161

CIRCULATING
PUMP  144

142A

174

155  142B

156

142

137

CONTROLLER

157  DRAIN TANK

# FIG.15 (not according to the invention)

# FIG.16 (not according to the invention)

```
                    ┌──────────┐
                    │  START   │
                    └────┬─────┘
                         │
       ┌─────────────────▼─────────────────┐
       │   START WATER-SOFTENING           │──── S21
       │      OPERATION MODE               │
       └─────────────────┬─────────────────┘
                         │
       ┌─────────────────▼─────────────────┐
       │   START FIRST TIMER (T1) COUNT    │──── S22
       └─────────────────┬─────────────────┘
                         │
        NO       ┌───────▼───────┐              S23
       ◄─────────┤ COUNT VALUE T1 ≧ A ?        │
                 └───────┬───────┘
                         │ YES
       ┌─────────────────▼─────────────────┐
       │   POLE CHANGE (POLE SWITCHING)    │──── S24
       └─────────────────┬─────────────────┘
                         │
       ┌─────────────────▼─────────────────┐
       │  START SECOND TIMER (T2) COUNT    │──── S25
       └─────────────────┬─────────────────┘
                         │
        NO       ┌───────▼───────┐              S26
       ◄─────────┤ COUNT VALUE T2 ≧ B ?        │
                 └───────┬───────┘
                         │ YES         S27
       ┌─────────────────▼─────────────────┐
       │     SWITCH THREE-WAY VALVE        │
       └─────────────────┬─────────────────┘
                         │
        NO       ┌───────▼───────┐              S28
       ◄─────────┤ COUNT VALUE T2 ≧ C          │
                 └───────┬───────┘
                         │ YES
```

FINISH THREE-WAY VALVE SWITCHING — S29

FINISH POLE CHANGE (POLE SWITCHING) — S30

RESET FIRST AND SECOND TIMERS (T1=0, T2=0) — S31

NO ◄── OPERATION FINISHED ? — S32
          │ YES
      ┌───▼───┐
      │  END  │
      └───────┘

# FIG.17 (not according to the invention)

<u>101</u>

# FIG.18 (not according to the invention)

```
                    ( START )
                        │
   ┌────────────────────┤
   │                    ▼
   │        ┌──────────────────────────┐
   │        │  START WATER-SOFTENING    │── S21
   │        │  OPERATION MODE           │
   │        └──────────────────────────┘
   │                    │
   │        ┌──────────────────────────┐
   │        │  START FIRST TIMER (T1) COUNT │── S22
   │        └──────────────────────────┘
   │                    │
   │         ┌──────────◄──────────┐
   │         │                     │
   │    NO   ▼                     S23
   │  ┌──◄◇ COUNT VALUE T1 ≧ A ? ◇
   │  │       │
   │  │       ▼ YES
   │  │  ┌──────────────────────────┐
   │  │  │  POLE CHANGE (POLE SWITCHING) │── S24
   │  │  └──────────────────────────┘
   │  │       │
   │  │  ┌──────────────────────────┐
   │  │  │  START SECOND TIMER (T2) COUNT │── S25
   │  │  └──────────────────────────┘
   │  │       │
   │  │    ┌──────◄──────┐
   │  │    │             S26
   │  │ NO ▼
   │  │ ◄◇ COUNT VALUE T2 ≧ B ? ◇
   │  │      │
   │  │      ▼ YES
   │  │ ┌──────────────────────────┐
   │  │ │  FINISH POLE CHANGE       │── S30
   │  │ │  (POLE SWITCHING)         │
   │  │ └──────────────────────────┘
   │  │      │
   │  │ ┌──────────────────────────┐
   │  │ │  RESET FIRST AND SECOND   │── S31
   │  │ │  TIMERS (T1=0, T2=0)      │
   │  │ └──────────────────────────┘
   │  │      │
   │  │      ▼              S32
   │  NO  ◇ OPERATION FINISHED ? ◇
   └─────◄   │
              ▼ YES
           ( END )
```

# FIG.19 (not according to the invention)

101

# FIG.20 (not according to the invention)

```
                    ┌─────────┐
                    │  START  │
                    └────┬────┘
                         ▼
          ┌──────────────────────────┐
          │  START WATER-SOFTENING    │── S21
          │     OPERATION MODE        │
          └────────────┬─────────────┘
                       ▼
          ┌──────────────────────────┐
          │ START FIRST TIMER (T1) COUNT │── S22
          └────────────┬─────────────┘
                       ▼                              S23
                  ╱─────────────╲
         NO      ╱ COUNT VALUE T1 ≧ A ? ╲
        ◄───────╲                       ╱
                 ╲─────────────────────╱
                       │ YES
                       ▼
          ┌──────────────────────────┐
          │ POLE CHANGE (POLE SWITCHING) │── S24
          └────────────┬─────────────┘
                       ▼              S25
          ┌──────────────────────────┐       ┌──────────────────────────┐  S34
          │ START SECOND TIMER (T2) COUNT │   │     OPEN DRAIN VALVE       │
          └────────────┬─────────────┘       └────────────┬─────────────┘
                       ▼              S26                  ▼              S35
                  ╱─────────────╲                    ╱─────────────╲
         NO      ╱ COUNT VALUE T2 ≧ B ? ╲    NO     ╱ COUNT VALUE   ╲
        ◄───────╲                       ╱   ◄──────╲   T2 ≧ D ?      ╱
                 ╲─────────────────────╱            ╲───────────────╱
                       │ YES     S30                      │ YES      S36
          ┌──────────────────────────┐       ┌──────────────────────────┐
          │    FINISH POLE CHANGE     │       │     CLOSE DRAIN VALVE     │
          │    (POLE SWITCHING)       │       └────────────┬─────────────┘
          └────────────┬─────────────┘                     ▼              S31
                       │          S33        ┌──────────────────────────┐
          ┌──────────────────────────┐       │  RESET FIRST AND SECOND   │
          │   CIRCULATING PUMP OFF    │       │  TIMERS (T1=0, T2=0)      │
          └────────────┬─────────────┘       └────────────┬─────────────┘
                       │ YES                               ▼              S32
                                             ╱─────────────╲
                                    NO      ╱   OPERATION    ╲
                                   ◄───────╲   FINISHED ?     ╱
                                            ╲───────────────╱
                                                  │ YES
                                             ┌─────────┐
                                             │   END   │
                                             └─────────┘
```

# FIG.21

(not according to the invention)

# FIG.22 (not according to the invention)

```
                    ┌─────────────┐
                    │    START    │
                    └──────┬──────┘
                           │
         ┌─────────────────▼──────────────────┐
         │  ┌──────────────────────────┐      │
         │  │   START WATER-SOFTENING   │──S21 │
         │  │      OPERATION MODE       │      │
         │  └─────────────┬─────────────┘      │
         │                ▼                    │
         │  ┌──────────────────────────┐       │
         │  │ START FIRST TIMER (T1) COUNT│─S22 │
         │  └─────────────┬─────────────┘       │
         │                ▼                     │
         │         ┌──────────────┐  S23        │
         │  NO     ◇ COUNT VALUE  ◇             │
         │ ◄───────◇  T1 ≧ A ?    ◇             │
         │         └──────┬───────┘             │
         │             YES│                     │
         │  ┌─────────────▼─────────────┐       │
         │  │ POLE CHANGE (POLE SWITCHING)│─S24  │
         │  └─────────────┬─────────────┘       │
         │                ▼                     │
         │  ┌──────────────────────────┐        │
         │  │START SECOND TIMER (T2) COUNT│─S25  │
         │  └─────────────┬─────────────┘        │
```

START WATER-SOFTENING OPERATION MODE — S21

START FIRST TIMER (T1) COUNT — S22

COUNT VALUE T1 ≧ A ? — S23 — NO / YES

POLE CHANGE (POLE SWITCHING) — S24

START SECOND TIMER (T2) COUNT — S25

COUNT VALUE T2 ≧ B ? — S26 — NO / YES

SWITCH THREE-WAY VALVE — S27

COUNT VALUE T2 ≧ C — S28 — NO / YES

FINISH THREE-WAY VALVE SWITCHING — S38

FINISH POLE CHANGE (POLE SWITCHING) — S30

RESET FIRST AND SECOND TIMERS (T1=0, T2=0) — S31

OPERATION FINISHED ? — S32 — NO / YES

END

53

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002181358 A
- JP 2007175140 A

- EP 1891982 A